# EUROPÄISCHE PATENTSCHRIFT

(11) **EP 0 998 900 B1**
(45) Veröffentlichungstag und Bekanntmachung des Hinweises auf die Patenterteilung: **03.03.2004**
(21) Anmeldenummer: 99119877.1
(22) Anmeldetag: 07.10.1999
(51) Int. Cl.: A61K 7/00, A61K 7/42

(54) **Verwendung von cholesterich-flüssigkristallinen Polymeren als UV-Filter in kosmetischen und pharmazeutischen Zubereitungen**
Use of cholesteric liquid crystal polymers as UV-filters in cosmetic and pharmaceutical preparations
Utilisation de polymères cristaux liquides de type cholesterique comme filtre UV dans des compositions cosmetiques et pharmaceutiques

(30) Priorität: 19.10.1998 DE 19848130
(43) Veröffentlichungstag der Anmeldung: 10.05.2000
(73) Patentinhaber: BASF AKTIENGESELLSCHAFT, 67056 Ludwigshafen (DE)
(72) Erfinder: Schumacher, Peter, Dr., 68163 Mannheim (DE); Prechtl, Frank, Dr., 60318 Frankfurt (DE)

(56) Entgegenhaltungen:
- EP-A- 0 962 222
- WO-A-93/22413
- FR-A- 2 586 693

## Beschreibung

Die Erfindung betrifft die Verwendung von cholesterisch-flüssigkristallinen Polymeren als photostabile UV-Filter in kosmetischen und pharmazeutischen Zubereitungen zum Schutz der menschlichen Epidermis oder menschlicher Haare gegen UV-Strahlung, speziell im Bereich von 280 bis 450 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Neben den bekannten UV-Absorbern, wie z.B. 4-Methoxy-zimtsäure-2-ethylhexylester oder 3-(4'-Methyl)-benzyliden-bornan-2-on werden in kosmetischen und pharmazeutischen Formulierugen häufig auch Lichtschutzmittel eingesetzt, die in Form von Pigmenten die UV-Strahlen reflektieren bzw. absorbieren. Die wichtigsten dieser verwendeten Pigmente sind Titandioxid und Zinkoxid. Bei hohen Einsatzkonzentrationen kann mit Pigmenten eine vollständige Abdeckung der Haut erreicht werden. Dann reflektieren die Partikel allerdings nicht nur UV-Strahlung sondern auch sichtbares Licht, was die häufig nicht gewünschte starke Eigenfärbung pigmenthaltiger Präparate bewirkt.

Während grobteilige Titandioxid-Pigmente (Teilchengröße > 500 nm) im UV-B- und UV-A-Bereich vergleichbar wirken, verschiebt sich das Wirkungsspektrum bei feinteiligem Material mit abnehmender Teilchengröße in Richtung UV-B. Dies zeigt, daß die Absorptions-/Reflektionscharakteristik direkt von der Größe und der Verteilung der Teilchen abhängt. Für einen ausgewogenen UV-B- und UV-A-Schutz sind daher bestimmte Teilchengrößenverteilungen erforderlich.

Von Nachteil bei der Verwendung der oben genannten Pigmente erweist sich, daß während der Lagerung der kosmetischen oder pharmazeutischen Lichtschutzmittelformulierungen oftmals Agglomeration, Aggregation und/oder Separation der Pigmentteilchen stattfinden. Die Folge der hierdurch veränderten optischen Eigenschaften kann eine drastisch verringerte Lichtschutzwirkung sein.

Als Alternative zu den o.g. Pigmenten beschreibt DE-A-196 19 460 die Verwendung von Flüssigkristallmischungen mit cholesterischer Phase enthaltend a) flüssigkristalline Organosiloxane, die Dianhydrohexit-Derivate als chirale Gruppen aufweisen und b) chirale monomere Zusatzstoffe, die die gleiche Helizität induzieren wie die jeweiligen flüssigkristallinen Organosiloxane, zur Herstellung von, für kosmetische Zwecke geeignete UV-Schutzschichten in Form von Filmen oder Plättchen. Die hier beschriebenen Flüssigkristallmischungen haben den Nachteil, daß sie sich aufgrund ihrer hohen Viskosität nur unbefriedigend zu Pigmenten verarbeiten lassen.

DE-A-196 29 761 beschreibt kosmetische oder pharmazeutische Zubereitungen, enthaltend Pigmente aus Polyorganosiloxanen mit vom Betrachtungswinkel abhängiger Farbigkeit. Bei den Pigmenten handelt es sich dabei um mindestens eine orientierte vernetzte Substanz einer flüssigkristallinen Struktur mit chiraler Phase. Die hier in den kosmetischen und pharmazeutischen Rezepturen offenbarten Pigmente besitzen zwar gewisse Absorptionseigenschaften im UV-Bereich. Sie haben jedoch für bestimmte Anwendungen den Nachteil, daß es sich hierbei um farbige Verbindungen handelt deren Einsatzgebiet dadurch eingeschränkt ist. Sehr häufig sind aber gerade solche kosmetischen und pharmazeutischen Zubereitungen gefragt, mit denen ein UV-Schutz erzielt wird, bei denen aber eine Färbung der Zubereitung unerwünscht ist.

Weitere, ebenfalls farbige, cholesterisch flüssigkristalline Polymere mit vom Betrachtungswinkel abhängigen Farbeffekten sind u.a. beschrieben in DE-A-196 31 658, DE-A-196 43 277, DE-A-196 12 973, DE-A-196 12 974, DE-A-196 12 975, DE-A-196 20 746 sowie in WO 97/14739.

Es bestand nun die Aufgabe, neue Lichtschutzmittel für kosmetische und pharmazeutische Zwecke vorzuschlagen, die im UV-A- und/oder UV-B-Bereich als Filter wirken und die in Form von Pigmenten die oben genannten Nachteile nicht aufweisen.

Diese Aufgabe wurde erfindungsgemäß gelöst durch die Verwendung von cholesterisch-flüssigkristallinen Polymeren als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

Bevorzugt sind die Polymere die aus folgenden Bausteinen der einzelnen Monomergruppen aufgebaut sind:
a) mindestens einen chiralen, bifunktionellen Molekülbaustein, mit dem eine cholesterisch-flüssigkristalline Phase mit einer Ganghöhe von kleiner 450 nm erzielt werden kann
   sowie mindestens eine weitere Moleküleinheit, die ausgewählt ist unter
b) achiralen Verbindungen aus der Gruppe der aromatischen Hydroxycarbonsäuren, cycloaliphatischen Hydroxycarbonsäuren, aromatischen Aminocarbonsäuren und cycloaliphatischen Aminocarbonsäuren;
c) achiralen Verbindungen aus der Gruppe der aromatischen Dicarbonsäuren und cycloaliphatischen Dicarbonsäuren und
d) achiralen Verbindungen aus der Gruppe der aromatischen Diole, cycloaliphatischen Diole, aromatischen Diamine und cycloaliphatischen Diamine.

Besonders bevorzugt sind cholesterische Hauptgruppenpolymere, enthaltend
a) 1 bis 60 mol-%, bevorzugt 3 bis 50 mol-% mindestens einer chiralen, bifunktionellen Moleküleinheit;
b) 0 bis 99 mol-%, bevorzugt 5 bis 90 mol-% mindestens einer achiralen Einheit aus der Gruppe der aromatischen Hydroxycarbonsäuren, cycloaliphatischen Hydroxycarbonsäuren, aromatischen Aminocarbonsäuren und cycloaliphatischen Aminocarbonsäuren;
c) 0 bis 49,5 mol-%, bevorzugt 0 bis 40 mol-% mindestens einer achiralen Einheit aus der Gruppe der aromatischen Dicarbonsäuren und cycloaliphatischen Dicarbonsäuren und
d) 0 bis 99 mol-%, bevorzugt 0 bis 49,5 mol-% mindestens einer achiralen Einheit aus der Gruppe der aromatischen Diole, cycloaliphatischen Diole, aromatischen Diamine und cycloaliphatischen Diamine,
e) 0 bis 5 mol-% einer verzweigbaren Komponente mit mehr als zwei funktionelle Gruppen,
wobei die Summe der Einzelkomponenten a) bis e) 100 mol-% ergibt.

Die Komponenten aus der Gruppe a) rekrutieren sich zweckmäßig aus dem chiralen pool". Hierunter wird in der Fachwelt (Ullmanns Encycl. Techn., 5. Auflage, Bd. A18, S. 183, 1991, VCH-Verlag) die Gesamtheit der natürlich vorkommenden chiralen Verbindungen verstanden. Insbesondere gehören hierzu sowohl die chiralen Bausteine tierischen als auch pflanzlichen Ursprungs. Dies schließt jedoch die Verwendung vollsynthetischer oder teilsynthetischer chiraler Molekülbausteine keinesfalls aus. So können auch aus Naturstoffen durch einen oder mehrere Syntheseschritte wertvolle chirale Komponenten erhalten werden, die so entweder nicht oder nur in geringen Mengen in der Natur vorkommen.

Insbesondere sind als Molekülbausteine a) generell alle chiralen, bifunktionellen Komponenten, beispielsweise chirale Diole oder Polyole, chirale Dicarbonsäuren, chirale Hydroxycarbonsäuren oder Aminocarbonsäuren gemeint.

Bevorzugte Vertreter aus der Gruppe a) sind folgende Verbindungen:

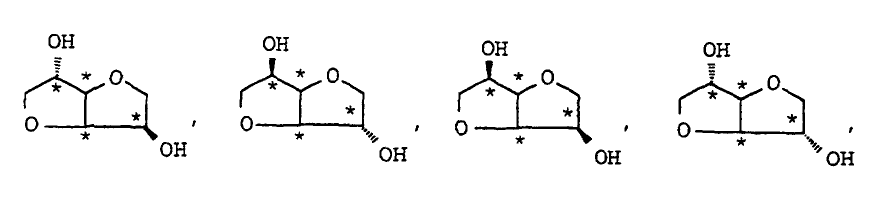

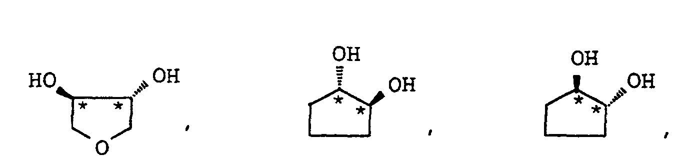

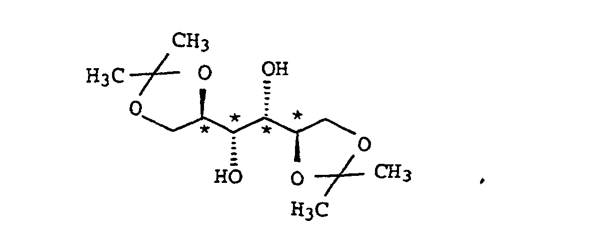

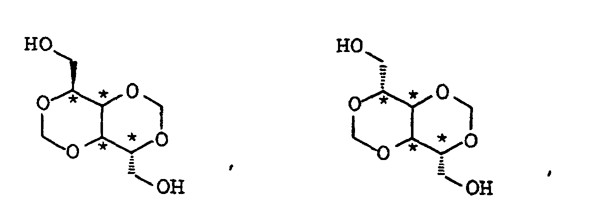

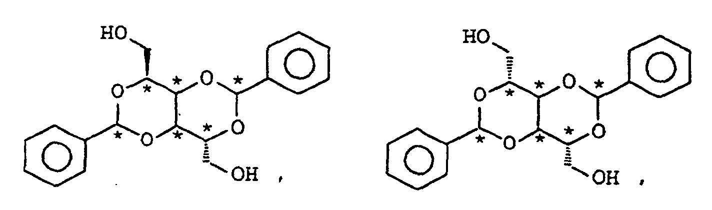

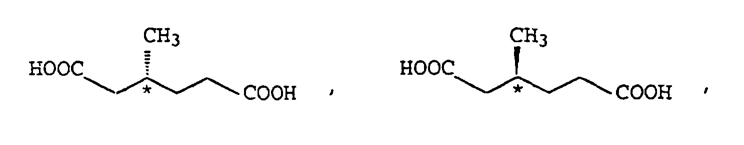

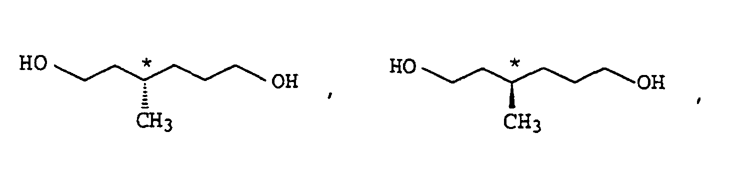

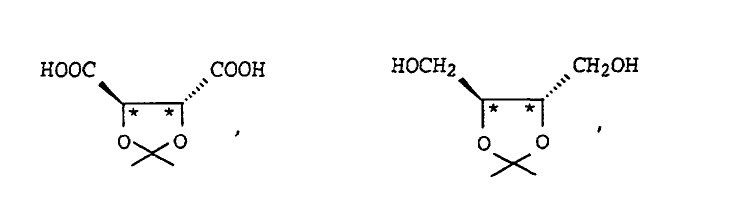

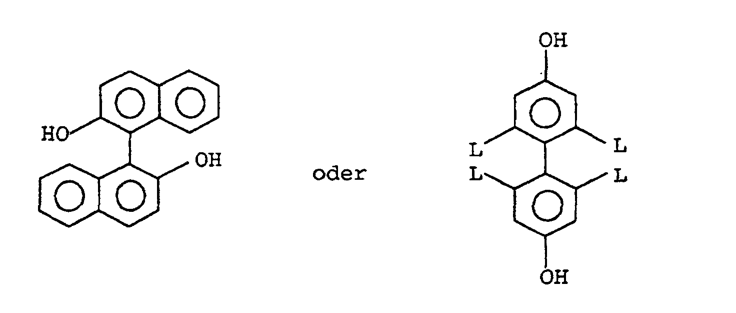

Besonders bevorzugte chirale, bifunktionelle Monomere sind:

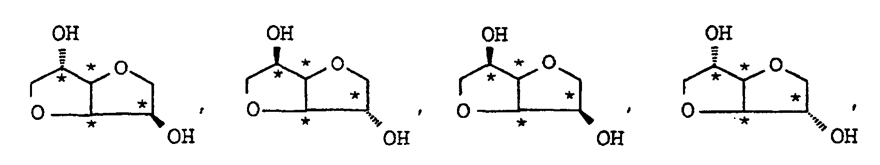

Die chiralen Comonomeren werden vorzugsweise in einer enantiomerenreinen Form eingesetzt. Bei Verwendung von Enantiomerengemischen eines Comonomeren ist darauf zu achten, daß eine Enantiomerenform in einem wirksamen Überschuß vorhanden ist.

Bevorzugte Komponenten b) sind:

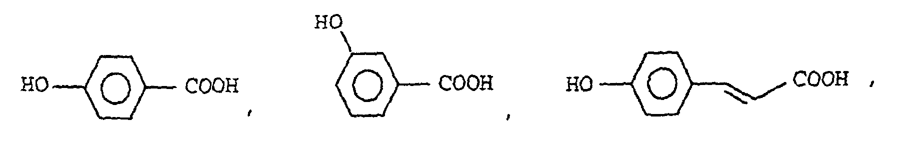

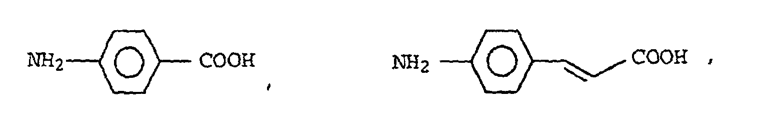

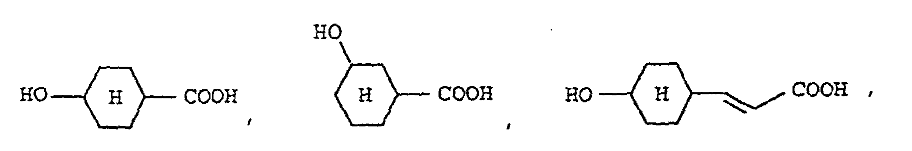

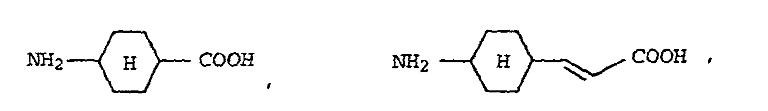

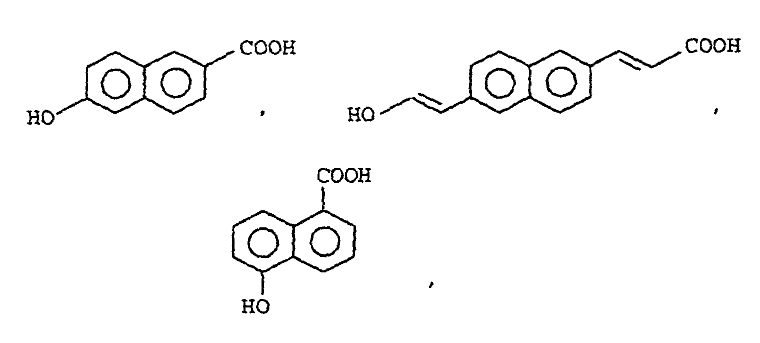

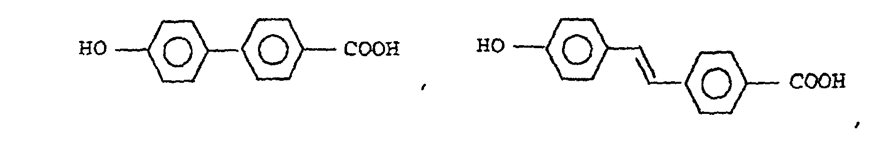

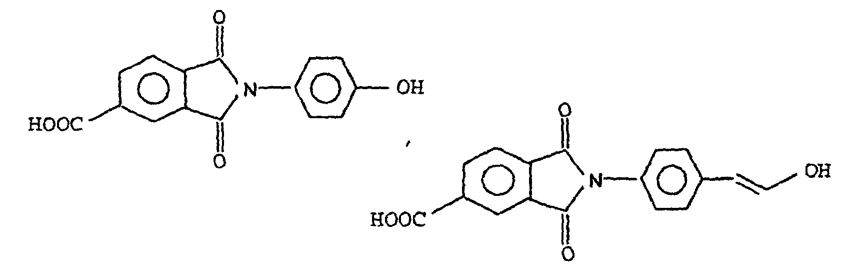

Besonders bevorzugte Vertreter aus der Gruppe b) sind:

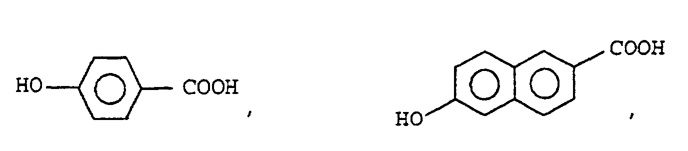

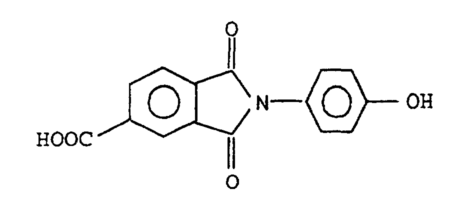

Bevorzugte Komponenten c) sind:

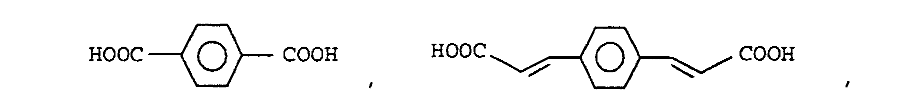

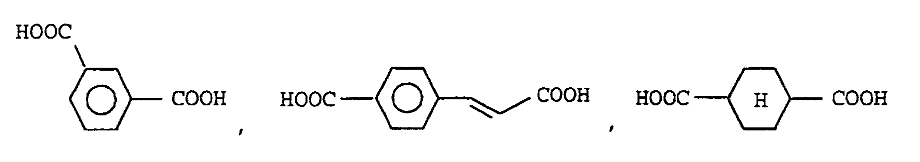

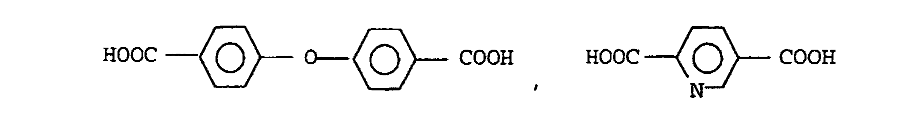

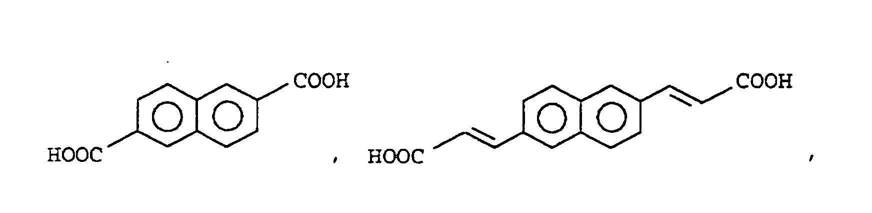

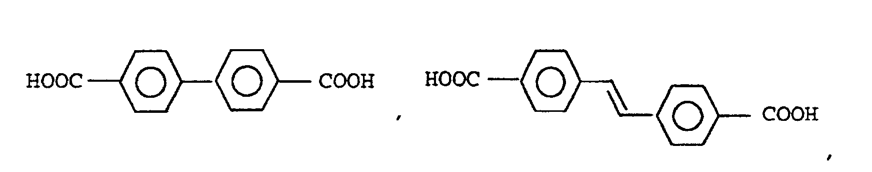

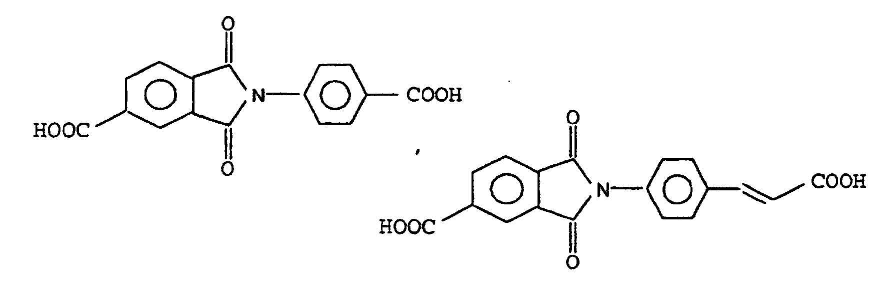

Besonders bevorzugte Vertreter aus der Gruppe c) sind:

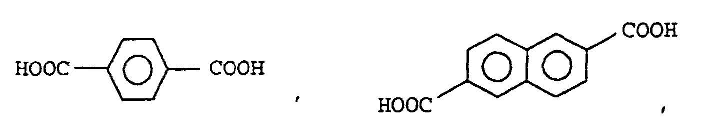

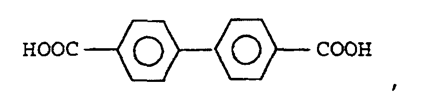

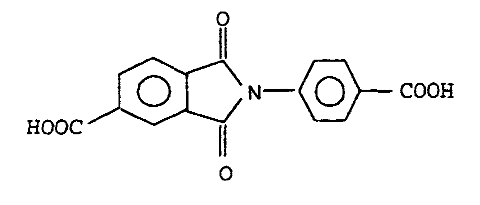

Bevorzugte Komponenten d) sind:

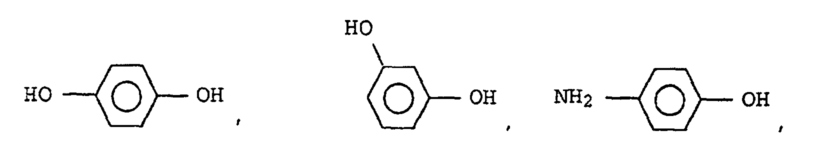

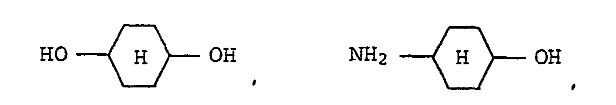

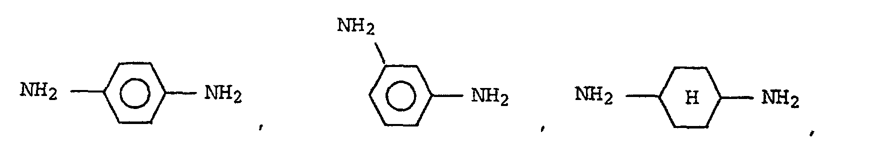

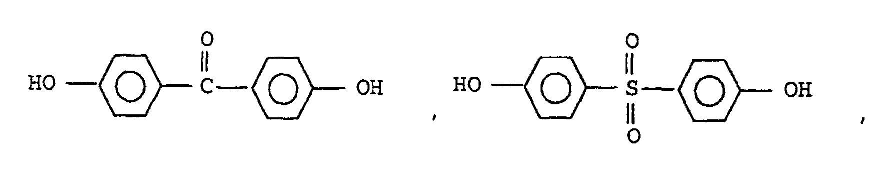

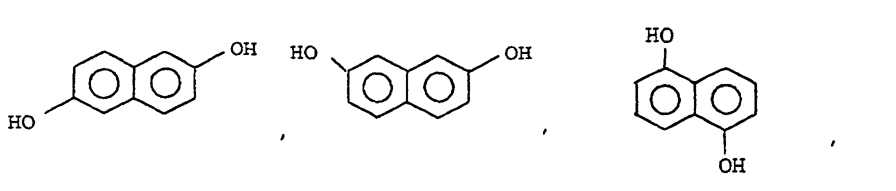

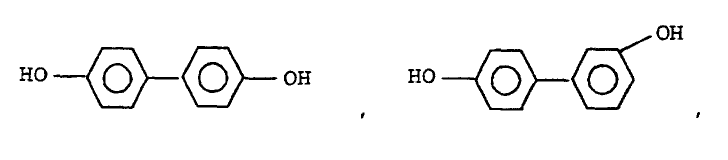

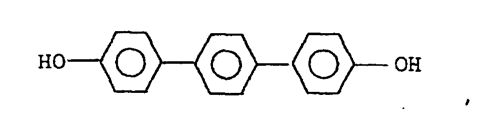

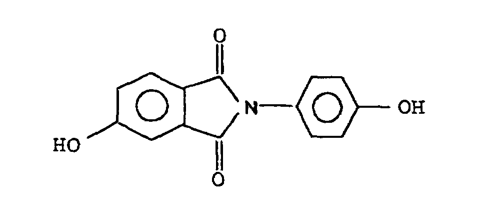

Besonders bevorzugte Vertreter aus der Gruppe d) sind:

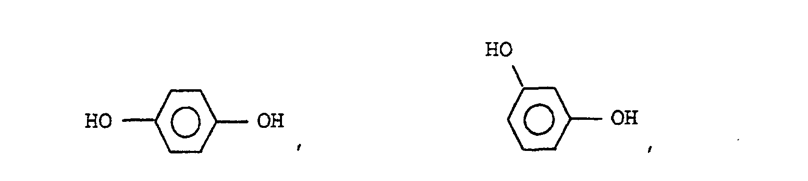

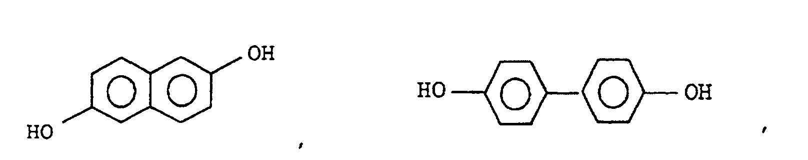

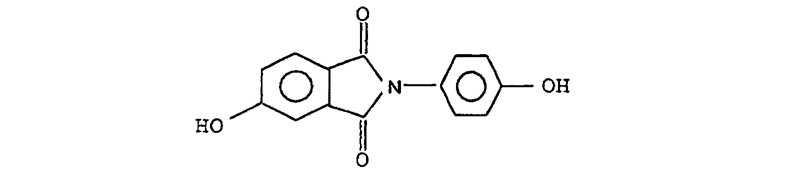

Anstelle der Carbonsäuren können auch andere dem Fachmann bekannte Carbonsäurederivate, wie beispielsweise Carbonsäurechloride, -anhydride oder -ester eingesetzt werden. Anstelle der Hydroxykomponenten können auch entsprechende Hydroxyderivate, wie z.B. die acylierten Hydroxyverbindungen eingesetzt werden.

Zusätzlich können die Polymere noch Komponenten mit mehr als zwei funktionellen Gruppen, wie beispielsweise Dihydroxybenzoesäure oder Trihydroxybenzole enthalten. Diese Komponenten wirken als Verzweigungsstelle im Polymer und werden gegebenenfalls nur in geringen Konzentrationen, beispielsweise 0 bis 5 mol-% zugegeben.

Die Herstellung der erfindungsgemäß verwendeten Polymeren kann durch verschiedene Arten der Polykondensation oder durch Umesterung von Polyestern mit einer Mischung aus Säurekomponente und acylierter Alkoholkomponente erfolgen.

Übliche Polykondensationsmethoden für die bevorzugt verwendeten Polyester sind beispielsweise das "HCl-Verfahren", das "Silyl-Verfahren" sowie das "Transesterifikationsverfahren". Diese Verfahren sind unter anderem aus H.R. Kricheldorf, N. Probst; Makromol. Rapid. Commun. (1995) 16, 231 und N. Probst, H.R. Kricheldorf; High Perform. Polym. (1995) 7, 461 bekannt.

Die Säurekomponenten und Alkoholkomponenten werden im molaren Verhältnis von ungefähr 1:1 eingesetzt.

Die Reaktionszeit kann in einem weiten Bereich variieren. Im allgemeinen beträgt sie 1 bis 48 h, insbesondere 1 bis 24 h.

Die Reaktion wird bei erhöhter Temperatur durchgeführt, im allgemeinen im Bereich von 120 °C bis 300 °C, wobei die Temperatur in diesem Bereich auch stufenweise erhöht werden kann.

Beim "HCl-Verfahren" werden die freien Diole mit den Dichloriden der Dicarbonsäuren in einem geeigneten organischen Lösungsmittel, z.B. einem Ether wie Dioxan, einem chlorierten Kohlenwasserstoff wie 1,1,2,2-Tetrachlorethan, 1-Chlornaphthalin oder 1,2-Dichlorbenzol, in einem geeigneten Reaktionsgefäß gelöst. Als Reaktionsgefäß geeignet ist beispielsweise ein druckfester Rührbehälter mit Gas-Ein- und Auslaßleitungen.

Vorzugsweise wird der freigesetzte Chlorwasserstoff im leichten Stickstoffstrom entfernt. Der gewonnene Polyester wird bei erhöhter Temperatur getrocknet, beispielsweise bei etwa 120°C im Vakuum. Gegebenenfalls wird der Polyester einem Reinigungsschritt durch erneutes Lösen in einem der obengenannten Lösungsmittel und Ausfällen in Methanol unterzogen.

Wenn das Polymer in dem verwendeten Lösungsmittel löslich ist, wird die organische Phase abgetrennt und der Polyester daraus in üblicher Weise gewonnen, z.B. durch Aufnehmen in Methanol und Abfiltrieren. Fällt das Polymer hingegen aus dem Lösungsmittel aus oder kommt es zur Gelbildung, wird das Reaktionsgemisch gegebenenfalls verdünnt, z.B. mit Methanol und das Polymer wird abfiltriert.

Beim "Silyl-Verfahren" werden die bissilylierten Diole mit den Dichloriden der Dicarbonsäuren in Anwesenheit einer katalytischen Menge eines quartären Ammoniumsalzes wie z.B. Triethylbenzylammoniumchlorid in Substanz oder mit einem der obengenannten Lösungsmittel in einem oben beschriebenen geeigneten Reaktionsgefäß erhitzt. Der erhaltene Polyester wird wie oben beschrieben aufgearbeitet.

Beim Transesterifikationsverfahren werden die freien Dicarbonsäuren mit den acetylierten Diolen in Gegenwart katalytischer Mengen eines Alkali- oder Erdalkalimetalloxids, (z.B. MgO) oder in Gegenwart von Zink-, Zinn-, Zirkon- Mangan-, und Wismut-Salzen in einem oben beschriebenen geeigneten Reaktionsgefäß in Substanz umgesetzt. Der erhaltene Polyester wird wie oben beschrieben aufgearbeitet.

Bei der Umesterung eines Polyesters (A) mit freier Dicarbonsäure (B) und acetyliertem Diol (C) werden die Reaktanden im Molmassenverhältnis A:B:C von etwa 1 bis 5 : 1 bis 5 : 1 bis 5, insbesondere etwa 1:2:2 eingesetzt. Die Reaktion wird in einem geeigneten Reaktionsgefäß vorzugsweise in Gegenwart katalytischer Mengen eines frühen Übergangsmetallalkylats, z.B. Titantetrabutylat, durchgeführt. Vorzugsweise wird das Reaktionsgefäß mehrere Male mit Stickstoff durchspült, um Luft zu entfernen. Die während der Reaktion freiwerdende Essigsäure wird vorzugsweise im leichten Stickstoffstrom entfernt. Die Reaktion wird vorzugsweise im Vakuum fortgeführt.

Von dem genannten Herstellungsverfahren sind das "HCl-Verfahren", das "Silyl-Verfahren" und die Umesterung besonders bevorzugt.

Die Herstellung der ebenfalls bevorzugten erfindungsgemäß verwendeten Polycarbonate mit Carbonateinheiten, die eine mesogene Gruppe umfassen und Carbonateinheiten, die eine chirale Gruppe umfassen kann durch verschiedene Arten der Polykondensation von Diolen aus der Gruppe a) und d) mit Phosgen oder Diphosgen erfolgen. Die Komponenten a) und d) werden im Molverhältnis von 1:1 bis 1:10000, bevorzugt 1:1 bis 1:1000 eingesetzt. Übliche Polykondensationsarten sind beispielsweise Grenzflächenpolykondensation, Schmelzpolykondensation und Lösungspolykondensation.

Bei der Grenzflächenpolykondensation werden die die mesogene Gruppe, die chirale Gruppe sowie gegebenenfalls eine photoreaktive Gruppe von Diolen zusammen mit Phosgen oder vorzugsweise dem wesentlich ungefährlicheren Diphosgen oder Triphosgen und einer katalytischen Menge eines Amins, beispielsweise Triethylamin oder eines quartären Ammoniumsalzes wie z.B. Triethylbenzylammoniumchlorid in einem geeigneten organischen Lösungsmittel, z.B. einem Ether wie Tetrahydrofuran oder Dioxan, einem chlorierten Kohlenwasserstoff, wie Dichlormethan oder Chlorbenzol, gelöst. Zu dieser Lösung wird eine wäßrige Base, z.B. Natriumhydroxidlösung gegeben und beide Phasen werden miteinander vermischt, z.B. durch heftiges Rühren. Während des Rührens wird vorzugsweise gekühlt. Wenn das Polymer in dem verwendeten Lösungsmittel löslich ist, wird die organische Phase abgetrennt und das Polycarbonat daraus in üblicher Weise gewonnen, z.B. durch Aufnehmen in Methanol und Abfiltrieren. Fällt das Polymer hingegen aus dem Lösungsmittel aus oder kommt es zur Gelbildung, wird das Reaktionsgemisch gegebenenfalls verdünnt, z.B. mit Methanol und das Polymer wird abfiltriert. Alternativ zu Phosgen oder Diphosgen können auch die chlorierten Kohlensäurediester der zu kondensierenden Diole eingesetzt werden.

Bei der Schmelzpolykondensation wird das Dicarbonat eines der die mesogene Gruppe, die chirale Gruppe sowie gegebenenfalls die photoreaktive Gruppe bildenden Diole mit den die restlichen Gruppen bildenden Diolen zur Reaktion gebracht. Die Reaktion wird bei höherer Temperatur durchgeführt, im allgemeinen im Bereich von 120°C bis 300°C, wobei die Temperatur in diesem Bereich auch stufenweise erhöht werden kann. Das erhaltene Polymer wird in einem der obengenannten geeigneten Lösungsmittel gelöst oder suspendiert und gegebenenfalls mit Methanol ausgefällt.

Bei der Lösungspolykondensation werden die die mesogene Gruppe, die chirale Gruppe sowie gegebenenfalls die weitere, nichtchirale Gruppe und die photoreaktive Gruppe bildenden Diole in einem Amin, vorzugsweise einem tertiären oder aromatischen Amin, beispielsweise Pyridin, gelöst. Zu dieser Lösung wird in einem der obengenannten geeigneten Lösungsmittel gelöstes Diphosgen gegeben. Die Reaktionstemperatur liegt im allgemeinen im Bereich von etwa 0°C bis Umgebungstemperatur, sie kann jedoch auch höher sein, insbesondere um die Reaktion zu vervollständigen. Das Gemisch wird dann in üblicher Weise aufgearbeitet. Alternativ zum Einsatz von Diphosgen können auch bei der Lösungspolykondensation die chlorierten Kohlensäurediester der zu polymerisierenden Diole verwendet werden.

Von den genannten Kondensationsmethoden sind die Grenzflächenpolykondensation und die Lösungspolykondensation bevorzugt, letztgenannte insbesondere dann, wenn hydrophilere Monomere, wie beispielsweise Isosorbid, eingesetzt werden, die bei der Grenzflächenpolykondensation wesentlich schlechter aus der wäßrigen in die organische Phase übergehen als die anderen Monomere mit denen sie kondensiert werden sollen.

Fallen die cholesterisch-flüssigkristallinen Polymere nicht bereits bei der Synthese als feinteiliges Pulver an, so müssen sie nach der Polymerisation in entsprechende feinteilige Pulver überführt werden.

Dazu bietet sich nach der Synthese als erster Zerkleinerungsschritt eine Strang- oder Bandextrusion an. Die dabei erhaltenen Stränge oder Bänder lassen sich in bekannter Weise mit Schnitzlern oder Granulatoren in Chips oder Stranggranulate überführen.

Zur weiteren Zerkleinerung bieten sich an sich bekannte Mahlaggregate aller Arten und Ausführungsformen an.

Für die erfindungsgemäße Verwendung der oben genannten cholesterisch-flüssigkristallinen Polymeren als UV-Filter in kosmetischen und pharmazeutischen Zubereitungen können diese direkt in die kosmetischen und pharmazeutischen Zubereitungen eingearbeitet werden.

Bevorzugt werden jedoch die erfindungsgemäß verwendeten cholesterisch-flüssigkristallinen Polymeren in Form von Pigmenten eingesetzt.

Zur Herstellung cholesterischer Pigmente gibt es mehrere Möglichkeiten:

Eine Möglichkeit, lösliche cholesterisch flüssigkristallinen Polymere in Pigmente zu überführen, ist die Lösungsbeschichtung. Hierbei wird das Polymer in einem Lösungsmittel gelöst und aus der Lösung als Film auf eine Unterlage, beispielsweise eine Folie, ein Metallband oder eine Metallwalze gebracht. Dies kann beispielsweise durch Spritzen, Rakeln, Gießen, Tauchen oder mit einem Pinsel geschehen. Nach dem Abdampfen des Lösemittels bildet das Polymer auf der Unterlage eine UV-reflektierende Schicht.

Eine weitere Möglichkeit, die cholesterisch flüssigkristallinen Polymere in Pigmente zu überführen, ist die Schmelzbeschichtung. Hierbei wird das Polymer in der Schmelze auf das Substrat aufgezogen oder auf dem Substrat aufgeschmolzen und zu einer dünnen Schicht verarbeitet.

Eine weitere Möglichkeit, die cholesterisch flüssigkristallinen Polymere in Pigmente zu überführen, stellt die Pulverbeschichtung dar. Hierbei wird das Polymer in einem Schritt mit bekannten Mahlaggregaten auf die gewünschte Kornfeinheit gemahlen. Anschließend wird das Pulver nach bekannten Methoden, wie beispielsweise Flammspritzverfahren appliziert.

Nach oder während des Auftragsprozesses auf die Unterlage wird die Pulverschicht auf Temperaturen oberhalb des Erweichungspunktes des Polymers erhitzt. Die Polymere bilden dabei einen homogenen Film, in dem sich die helikalen Überstrukturen ausbilden. Die Temperatur, bei welcher die Ausbildung der helikalen Struktur beginnt, wird im folgenden als Chiralisierungstemperatur bezeichnet.

Die Herstellung von UV-Pigmenten kann z.B. durch Ablösen des orientierten Polymerfilms von der beschichteten Oberfläche und Mahlung zu plättchenförmigen Pigmenten sowie durch entsprechende Mahlung der extrudierten Polymerstränge erfolgen.

Die speziellen UV-Licht reflektierenden Eigenschaften der erfindungsgemäß verwendeten Pigmente werden erst beobachtet, wenn die Moleküle oberhalb der Chiralisierungstemperatur des Polymers die helikalen Strukturen ausbilden. Der Übergang in die cholesterische Phase erfolgt in vielen Fällen bereits bei der Synthese der Polymeren. Die Wellenlänge der Selektivreflektion der erfindungsgemäß verwendeten Polymere wird durch die Ganghöhe der helikalen Struktur bestimmt. Die Ganghöhe ist abhängig von der Struktur der Polymeren und von der Verdrillungskraft des chiralen Monomeren. Sie ist außerdem eine Funktion der Temperatur. Entsprechend läßt sich die Ganghöhe der Helix auch über die Temperatur einstellen. Durch schnelles Abkühlen der beschichteten Substrate läßt sich die Ganghöhe der Helix und somit die Selektivreflexion dauerhaft einfrieren. Für die Anwendung in der Praxis ist es wichtig, daß der Schmelzpunkt und die Chiralisierungstemperatur des Polymeren oberhalb der Gebrauchstemperatur des Pigmentes liegen.

Für den Fall, daß das Polymer photovernetzbare Gruppen enthält, kann die UV-reflektierende Struktur des Polymers auch durch photochemische Vernetzung der chiral nematischen Phase fixiert werden.

Je nach der erwünschten Anwendung bzw. je nach Art der kosmetischen oder pharmazeutischen Formulierung können Korngrößen mit einem Durchmesser von 1 bis 1000 µm hergestellt werden. Bevorzugte Korngrößen liegen im Bereich zwischen 1 und 100 µm, besonders bevorzugt zwischen 15 und 50 µm.

Die Dicke der Pigmente liegt zwischen 1 und 100 µm, bevorzugt zwischen 1 und 50 µm besonders bevorzugt zwischen 1,5 und 10 µm.

Einen Überblick über Verfahren, orientierte Ausgangsstoffe photochemisch zu vernetzen, findet sich bei C.G. Roffey, Photopolymerisation of Surface Coatings, (1982) John Willey & Sons, Chichester, S. 137 bis 208.

Die cholesterisch-flüssigkristallinen Polymeren, die als Ausgangssubstanzen zur Herstellung der Pigmente geeignet sind, besitzen eine verdrillte Struktur mit einer Ganghöhe, die einer Wellenlänge des Lichtes bis zu 450 nm entspricht. Art und Anteil der chiralen Substanz bestimmen die Ganghöhe der verdrillten Struktur und damit die Wellenlänge des reflektierten Lichtes. Je nach Chiralität der eingesetzten optisch aktiven Zusatzstoffe kann die Verdrillung der Struktur sowohl links- als auch rechtsgängig sein.

Sogenannte Breitbandreflektoren lassen sich durch einfaches Mischen mehrerer der erfindungsgemäß zu verwendenden cholesterisch flüssigkristallinen Pigmente mit jeweils unterschiedlichen UV-Reflektionsmaxima erzeugen.

Darüber hinaus ist es möglich, durch Mischen von mindestens zwei verschiedenen Pigmenten der cholesterisch-flüssigkristallinen Polymeren mit jeweils entgegengesetzter Verdrillung (Helicität) eine vollständige Reflektion der UV-Strahlen zu erzielen. Pigmente solcher jeweils entgegengesetzt verdrillten, cholesterisch-flüssigkristallinen Strukturen sind beispielsweise durch Zugabe jeweils der einzelnen Spiegelbildisomeren (Enantiomeren) oder Diastereomeren der chiralen Monomeren a) zu den achiralen polymerisierbaren Monomeren b-d) erhältlich. Die Ganghöhe der jeweils entgegengesetzt verdrillten Strukturen kann dabei gleich oder verschieden sein.

Es ist auch möglich, zunächst die cholesterisch-flüssigkristallinen Polymeren von jeweils entgegengesetzter Verdrillung zu mischen, diese anschließend in die bereits beschriebenen Pigmente zu überführen und als UV-Reflektoren in kosmetischen und pharmazeutischen Formulierungen einzusetzen.

Neben den o.g. Mischungen cholesterisch flüssigkristalliner Pigmente ist es auch möglich Mehrschichtpigmente zu erzeugen, deren einzelne Schichten verschiedene, erfindungsgemäß zu verwendende, cholesterisch flüssigkristalline Polymeren enthalten. Das Design derartiger Mehrschichtpigmente läßt sich vielfältig variieren. So können u.a.
- einzelne Schichten von cholesterisch flüssigkristallinen Polymeren entgegengesetzter Verdrillung oder
- einzelne Schichten von cholesterisch flüssigkristallinen Polymeren gleicher Gangrichtung aber unterschiedlicher Ganghöhe und somit unterschiedlicher Reflektionseigenschaften
übereinander aufgebracht werden.

Bevorzugt sind sogenannte Dreischichtpigmente, bei denen die beiden äußeren Schichten aus jeweils einer der erfindungsgemäß zu verwendenden, cholesterisch flüssigkristallinen Polymeren besteht und die mittlere Schicht beispielsweise eine Bindemittelmatrix enthalten kann, in dem zusätzlich ein weiterer UV-Absorber eingearbeitet sein kann. Einzelheiten bezüglich Herstellung, Eigenschaften und weiterer Bestandteile solcher mehrschichtigen cholesterischen Pigmente sind der deutschen Patentanmeldung P 19738368.8 zu entnehmen.

Besonders bevorzugt sind Zweischichtpigmente, bei denen die beiden Schichten aus jeweils einer der erfindungsgemäß zu verwendenden, cholesterisch flüssigkristallinen Polymeren besteht und die durch Coextrusion hergestellt werden.

Gegenstand der Erfindung sind somit auch die oben beschriebenen Pigmente, insbesondere Mehrschichtpigmente, enthaltend die eingangs genannten cholesterisch-flüssigkristallinen Polymeren.

Ein Vorteil der erfindungsgemäß verwendeten Pigmente liegt darin, daß deren Zusammensetzung so maßgeschneidert eingestellt werden kann, damit mit diesen Pigmenten die gewünschte UV-Reflektion erzielt werden kann ohne aber eine eigene Farbigkeit (im sichtbaren Bereich) zu zeigen.

Ein weiterer Vorteil der Pigmente liegt in ihren physikalischen Eigenschaften. Aufgrund ihrer geringen Dichte (im Vergleich beispielsweise zu TiO₂) lassen sich die Pigmente gut in Emulsionen einarbeiten, ohne daß es zu Aggregation oder Separation der Pigmentteilchen kommt.

Die erfindungsgemäß zu verwendenden Pigmente lassen sich durch einfaches Abmischen in die kosmetischen und pharmazeutischen Zubereitungen einarbeiten.

Gegenstand der vorliegenden Erfindung sind weiterhin kosmetische und pharmazeutische Zubereitungen, die 0,1 bis 20 Gew.-%, vorzugsweise 0,5 bis 10 Gew.-%, besonders bevorzugt 1 bis 7 Gew.-%, bezogen auf die gesamte Menge der kosmetischen und pharmazeutischen Zubereitung zusammen mit an sich für kosmetische und pharmazeutische Zubereitungen bekannten, im UV-A- und UV-B-Bereich absorbierenden Verbindungen als Lichtschutzmittel enthalten. Die Polymeren sowie die Stoffklasse der eingesetzten chiralen und achiralen Monomere entsprechen dabei sowohl in ihrer allgemeinen als auch in ihrer bevorzugten Ausführungsform den bereits oben geschilderten Erläuterungen.

Bevorzugt sind solche der oben genannten kosmetischen und pharmazeutischen Zubereitungen, die die erfindungsgemäß zu verwendenden cholesterisch-flüssigkristallinen Polymere in Form der bereits beschriebenen Pigmente, insbesondere in Form von mehrschichtigen Pigmenten enthalten.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasserin-Öl-Cremes, Öl-in-Wasser-Cremes und Lotionen, Aerosol-Schaumcremes, Gele, Öle, Fettstifte, Puder, Sprays oder alkoholischwäßrige Lotionen.

Übliche Ölkomponenten in der Kosmetik sind beispielsweise Paraffinöl, Glycerylstearat, Isopropylmyristat, Diisopropyladipat, 2-Ethylhexansäurestearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminiumund/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Gegebenenfalls können die erfindungsgemäßen Zubereitungen zusätzlich ein oder mehrere Antioxidantien enthalten. Als günstige, aber dennoch fakultativ zu verwendende Antioxidantien können alle für kosmetische und/oder dermatologische Anwendungen geeigneten oder gebräuchlichen natürlichen, synthetischen und/oder partialsynthetischen Antioxidantien verwendet werden.

Besonders vorteilhaft werden die Antioxidantien gewählt aus der Gruppe, bestehend aus:

Aminosäuren (z.B. Glycin, Histidin, Tyrosin, Tryptophan) und deren Derivate, Imidazole (z.B. Urocaninsäure) und deren Derivate, Peptide wie D,L,-Carnosin, D-Carnosin, L-Carnosin und deren Derivate (z.B. Anserin), Carotinoide (z.B. β-Carotin, Lycopin) und deren Derivate, Liponsäure und deren Derivate (z.B. Dihydroliponsäure), Aurothioglucose, Propylthiouracil und andere Thioverbindungen (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA, EGTA und deren Derivate, ungesättigte Fettsäuren und deren Derivate (z.B. γ-Linolensäure, Linolsäure, Ölsäure), Folsäure und deren Derivate (z.B. 5-Methyltetrahydrofolsäure), Ubichinon und Ubichinol und deren Derivate, Vitamin C und deren Derivate (z.B. Ascorbylpalmitat, Ascorbylphosphate, Ascorbylacetate), Tocopherole und Derivate (z.B. Tocopherylacetat, Tocotrienol), Vitamin A und Derivate (z.B. Vitamin A-Palmitat), Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Stilbene und deren Derivate.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Zubereitung - betragen. Die Herstellung der Zubereitung kann in an sich bekannter Weise, d.h. beispielsweise durch Heiß-, Kalt-, Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden cholesterisch-flüssigkristallinen Zusammensetzungen 10 bis 90 Gew.-%, bevorzugt 20 bis 70 Gew.-% bezogen auf die Gesamtmenge von UV-B und UV-A absorbierenden Substanzen.

Als UV-Filtersubstanzen, die in Kombination mit den erfindungsgemäß zu verwendenden cholesterisch-flüssigkristallinen Zusammensetzungen angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]-heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure (Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Methyl-o-aminobenzoate oder: | 134-09-8 |
| | 5-Methyl-2-(1-methylethyl)-2-aminobenzoate | |
| 22 | Glyceryl p-aminobenzoat oder: | 136-44-7 |
| | 4-Aminobenzoesäure-1-glyceryl-ester | |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: | 4732-70-1 |
| | 3,4-dimethoxy-phenyl-glyoxal-saures Natrium | |
| 27 | 3-(4'Sulfo)benzyliden-bornan-2-on und seine Salze | 56039-58-8 |
| 28 | 4-tert.-Butyl-4-methoxy-dibenzoylmethan | 70356-09-1 |
| 29 | 2,2',4,4-Tetrahydroxybenzophenon | 131-55-5 |
| 30 | Dimethicon-diethylbenzalmalonat | 207574-74-1 |
| 31 | Bis[2-hydroxy-5-tert.-octyl-3-(benzotriazol-2-yl)phenyl]methan (Bisoctyltrialon) | 103597-45-1 |
| 32 | 1H-Benzimidazol-4,6-disulfonsäure, 2,2'-(1,4-phenylen)bis-Dinatriumsalz (Benzimidazylat) | 180898-37-7 |
| 33 | Phenol, 2,2'-[6-(4-methoxyphenyl)-1,3,5-triazin-2,4-diyl]bis[5-[(2-ethylhexyl)oxy] (Aniso Triazin) | 187393-00-6 |

Schließlich sind auch mikronisierte Pigmente wie Titandioxid und Zinkoxid zu nennen.

Zum Schutz menschlicher Haare vor UV-Strahlen können die erfindugsgemäß verwendeten cholesterisch flüssigkristallinen Polymere in Shampoos, Lotionen, Gelen, Haarsprays, Aerosol-Schaumcremes oder Emulsionen in Konzentrationen von 0,1 bis 20 Gew.-%, bevorzugt 0,5 bis 10 Gew.-%, besonders bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Zusammensetzungen zeichnen sich in der Regel durch ein besonders hohes Reflektionsvermögen im Bereich der UV-A- und UV-B-Strahlung mit scharfer Bandenstruktur aus. Weiterhin lassen sie sich leicht in kosmetische und pharmazeutischen Formulierungen einarbeiten. Außerdem zeichnen sie sich besonders durch ihre hohe Photostabilität, und die damit hergestellten Zubereitungen durch ihr angenehmes Hautgefühl aus.

Die UV-Filterwirkung der erfindungsgemäßen verwendeten cholesterisch flüssigkristallinen Polymeren kann auch zur Stabilisierung von Wirk- und Hilfsstoffen in kosmetischen und pharmazeutischen Formulierungen ausgenutzt werden.

Anhand der folgenden Beispiele soll der Gegenstand der vorliegenden Erfindung näher erläutert werden.

### Beispiel 1

19665 Teile 2-Hydroxy-6-naphthoesäure, 24067 Teile 4-Hydroxybenzoesäure, 18343 Teile Terephthalsäure, 93 Teile 4,4'-Dihydroxybiphenyl und 16060 Teile 1,4:3,6-Dianhydro-D-sorbit (Isosorbid) wurden in einem Reaktor mit 52680 Teilen Essigsäureanhydrid versetzt und mit einem leichten Stickstoffstrom durschpült. Die Mischung wurde unter Rühren innerhalb von 15 min auf 140°C erhitzt und diese Temperatur 30 min gehalten. Danach wurde die Temperatur innerhalb von 165 min auf 325°C erhöht und die Schmelze 30 min bei dieser Temperatur weiter gerührt. Ab ca. 220°C begann Essigsäure abzudestillieren. Danach wurde die Stickstoffspülung abgebrochen und langsam Vacuum angelegt. Die Schmelze wurde für weitere 30 min unter Vacuum (ca. 5 mbar) gerührt. Danach wurde mit Stickstoff belüftet und das Polymer mit einem Extruder ausgetragen und pelletiert. Remissionsmessungen zeigten eine Bande im UV-Bereich.

### Zubereitungen

### Beispiel 2

| Zusammensetzung für die Lippenpflege | |
|---|---|
| Massengehalt (Gew.-%) | |
| ad 100 | Eucerinum anhydricum |
| 10,00 | Glycerin |
| 5,00 | Pigment von Polymer aus Beispiel 1 |
| 8,00 | Octyl Methoxycinnamat |
| 5,00 | Zink Oxid |
| 4,00 | Castoröl |
| 4,00 | Pentaerythrithyl Stearat/Caprat/Caprylat Adipat |
| 3,00 | Glyceryl Stearat SE |
| 2,00 | Bienenwachs |
| 0,50 | Tocopheryl Acetat |
| 2,00 | Microkristallines Wachs |
| 2,00 | Quaternium-18 Bentonit |
| 1,50 | PEG-45/Dodecyl Glycol Copolymer |

### Beispiel 3

| Zusammensetzung für Sunblocker mit Mikropigmenten | |
|---|---|
| Massengehalt (Gew.-%) | |
| ad 100 | Wasser |
| 10,00 | Octyl Methoxcinnamat |
| 6,00 | PEG-7-Hydrogenated Castor Öl |
| 6,00 | Titanium Dioxid |
| 5,00 | Pigment von Polymer aus Beispiel 1 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 4

| Fettfreies Gel | |
|---|---|
| Massengehalt (Gew.-%) | |
| ad 100 | Wasser |
| 8,00 | Octyl Methoxycinnamat |
| 5,00 | Pigment von Polymer aus Beispiel 1 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 5

| Sonnencreme (LSF 20) | |
|---|---|
| Massengehalt (Gew.-%) | |
| ad 100 | Wasser |
| 8,00 | Octyl Methoxycinnamat |
| 6,00 | PEG-7-Hydrogenated Castor Öl |
| 5,00 | Pigment von Polymer aus Beispiel 1 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 6

| Sonnencreme wasserfest | |
|---|---|
| Massengehalt (Gew.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Pigment von Polymer aus Beispiel 1 |
| 4,00 | Glycerin |
| 3,00 | Jojoba Öl |
| 2,00 | 4-Methylbenzyliden Campher |
| 1,50 | PEG-45/Dodecyl Glycol Copolymer |
| 1,50 | Dimethicon |
| 0,70 | Magnesium Sulfat |
| 0,50 | Magnesium Stearat |
| 0,50 | Tocopheryl Acetat |
| 0,15 | Fragrance |

### Beispiel 7

| Sonnenmilch (LSF 6) | |
|---|---|
| Massengehalt (Gew.-%) | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 3,00 | Pigment von Polymer aus Beispiel 1 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |

## Patentansprüche

1. Verwendung von cholesterisch-flüssigkristallinen Polyestern, Polycarbonaten oder Polyamiden, enthaltend
a) mindestens einen chiralen, bifunktionellen Molekülbaustein, ausgewählt aus der Gruppe, bestehend aus 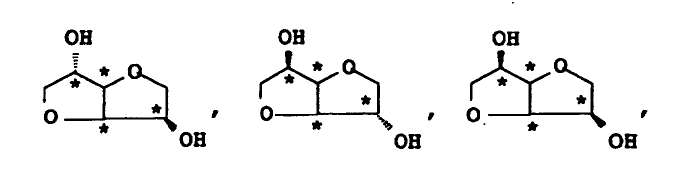 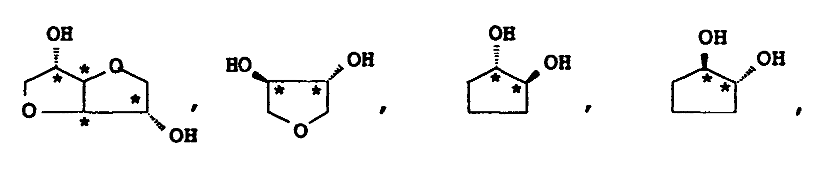 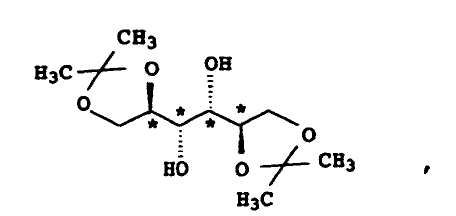 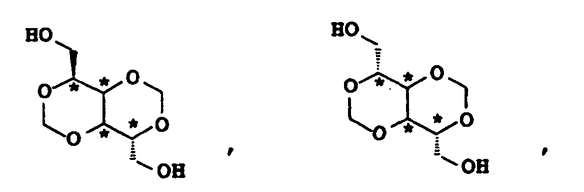 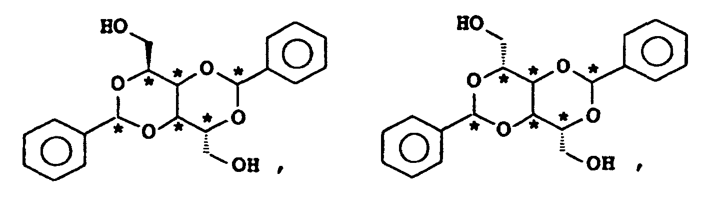 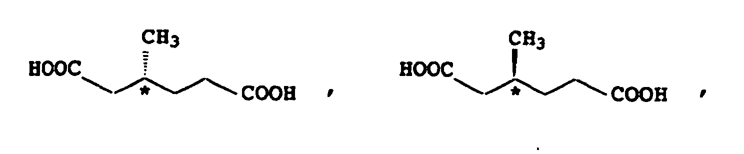 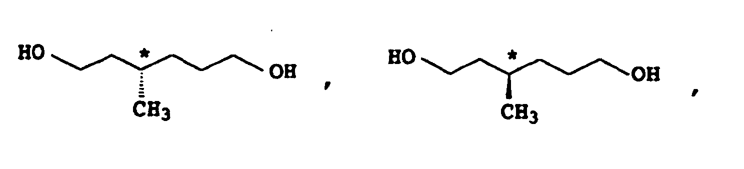 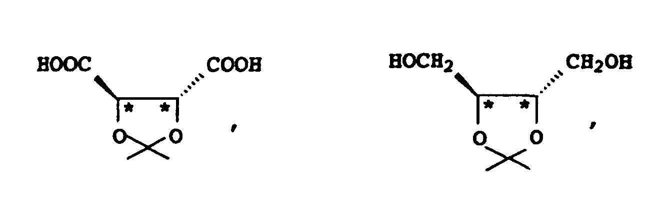 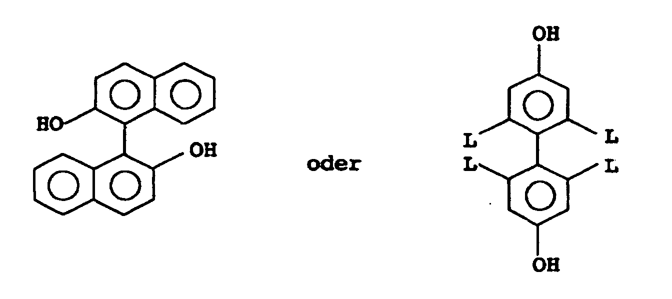 mit dem eine cholesterisch-flüssigkristalline Phase mit einer Ganghöhe von kleiner 450 nm erzielt werden kann, sowie mindestens eine weitere Moleküleinheit, die ausgewählt ist unter
b) achiralen Verbindungen aus der Gruppe der aromatischen Hydroxycarbonsäuren, cycloaliphatischen Hydroxycarbonsäuren, aromatischen Aminocarbonsäuren und cycloaliphatischen Aminocarbonsäuren, bestehend aus 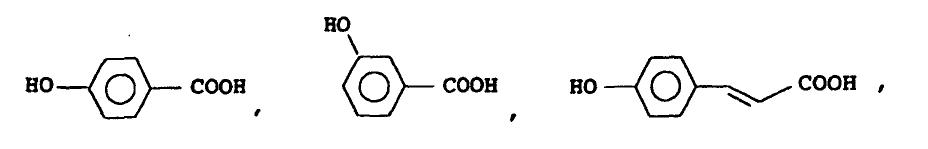 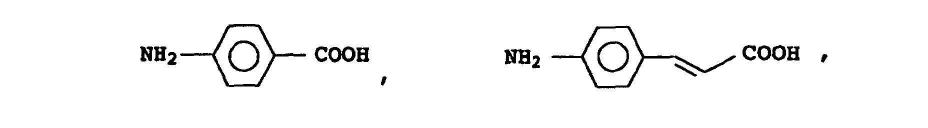 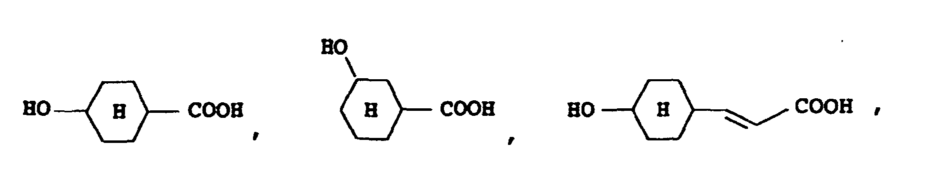 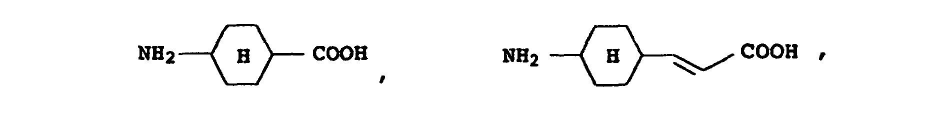 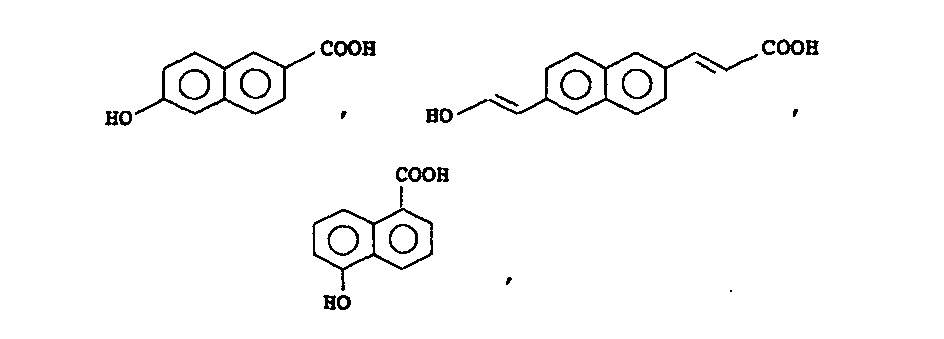 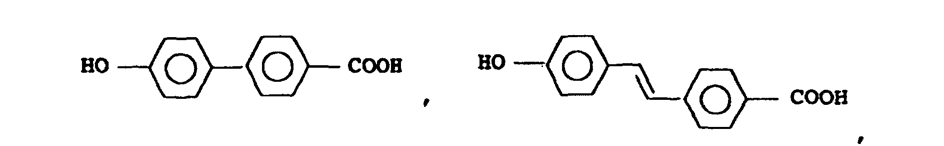 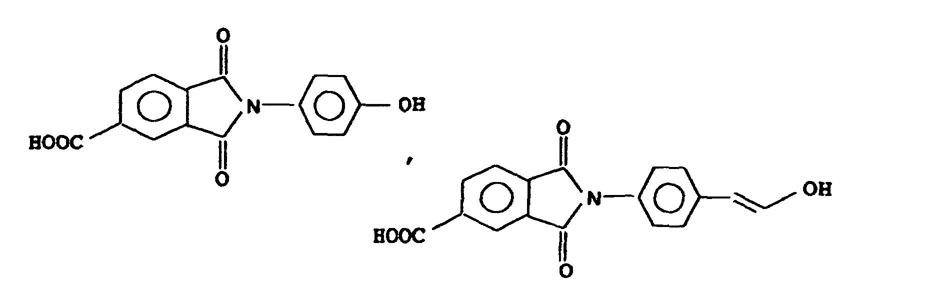
c) achiralen Verbindungen aus der Gruppe der aromatischen Dicarbonsäuren und cycloaliphatischen Dicarbonsäuren, bestehend aus 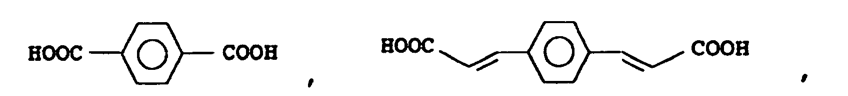 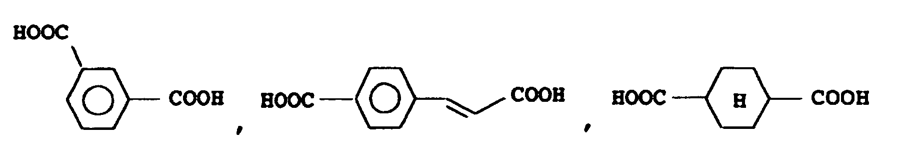 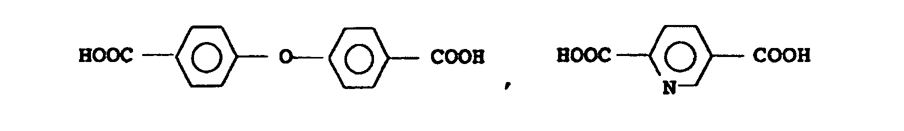 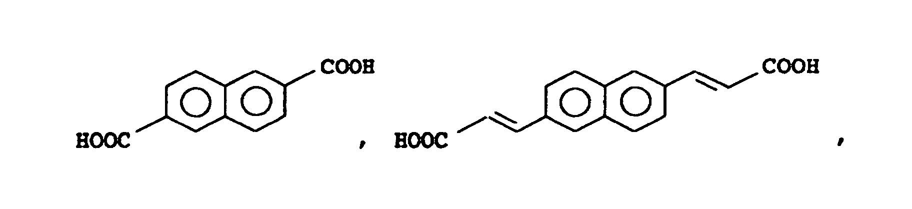 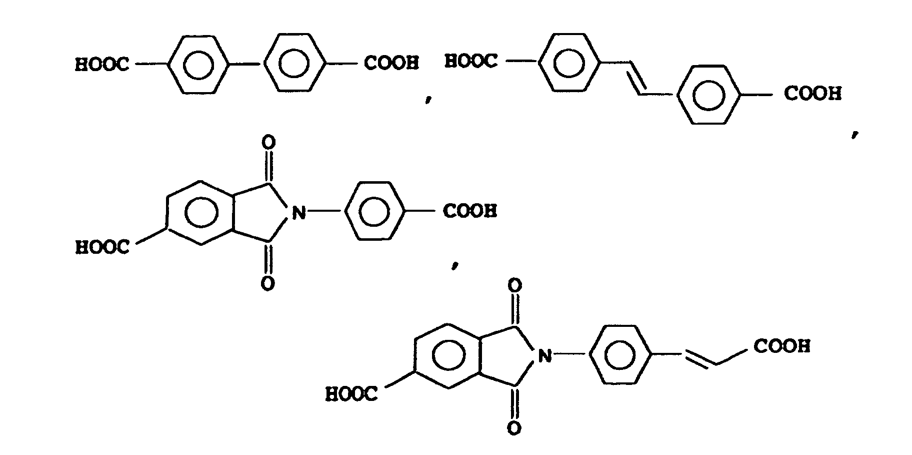 und
d) achiralen Verbindungen aus der Gruppe der aromatischen Diole, aromatischen Diamine und cycloaliphatischen Diamine, bestehend aus 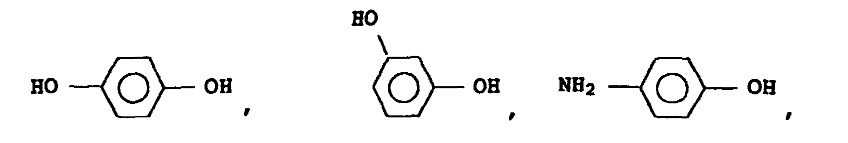 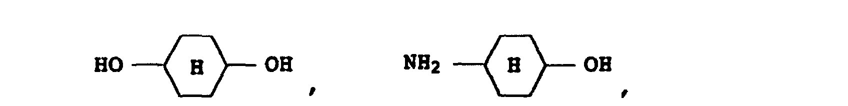 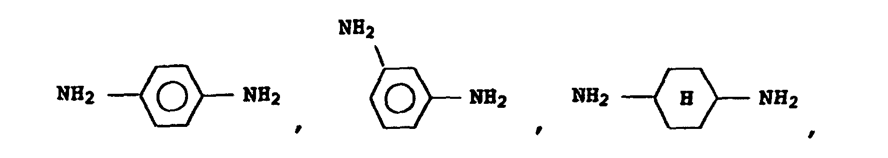 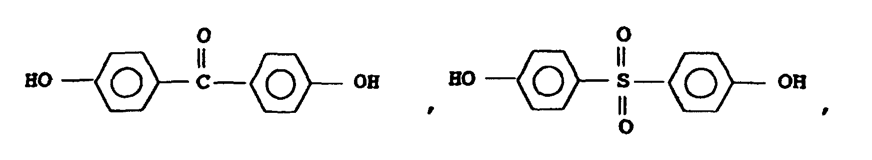 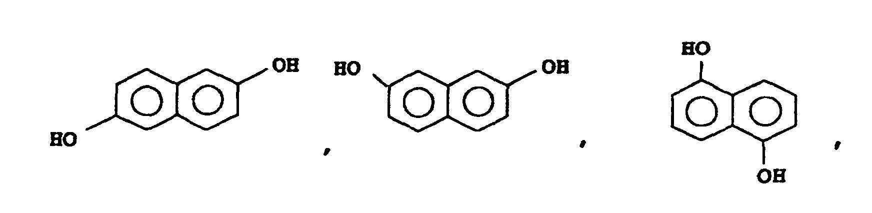 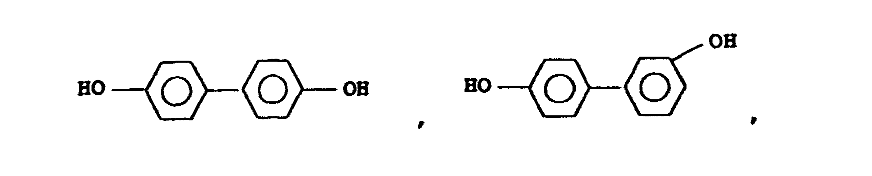 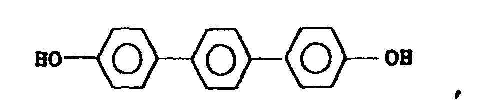 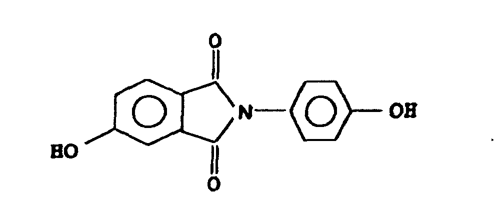 als UV-Filter in kosmetischen und pharmazeutischen Zubereitungen zum Schutz der menschlichen Haut oder menschlichen Haare gegen Sonnenstrahlen, allein oder zusammen mit an sich für kosmetische und pharmazeutische Zubereitungen bekannten, im UV-Bereich absorbierenden Verbindungen.

2. Verwendung von cholesterisch-flüssigkristallinen Polymeren nach Anspruch 1, enthaltend
1 bis 60 Mol-% mindestens eines der chiralen, bifunktionellen Molekülbausteine a;
0 bis 99 Mol-% mindestens einer achiralen Verbindung b;
0 bis 49,5 Mol-% mindestens einer achiralen Verbindung c;
0 bis 99 Mol-% mindestens einer achiralen Verbindung d und
0 bis 5 Mol-% Dihydroxybenzoesäure oder Trihydroxybenzole e,
wobei die Summe der Einzelkomponenten a) bis e) 100 Mol-% ergibt.

3. Verwendung von cholesterisch-flüssigkristallinen Polymeren nach einem der Ansprüche 1 oder 2 als photostabile UV-Reflektoren.

4. Verwendung von cholesterisch-flüssigkristallinen Polymeren definiert gemäß Anspruch 1 als UV-Stabilisatoren in kosmetischen und pharmazeutischen Formulierungen.

5. Verwendung von cholesterisch-flüssigkristallinen Polymeren nach einem der Ansprüche 1 bis 4 in Form von Pigmenten.

6. Pigmente enthaltend cholesterisch-flüssigkristalline Polymeren, definiert gemäß Anspruch 1.

7. Pigmente nach Anspruch 6, **dadurch gekennzeichnet, daß** es sich um Mehrschichtpigmente handelt.

8. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische zubereitungen bekannten im UV-Bereich absorbierenden Verbindungen, als photostabile UV-Filter wirksame Mengen von cholesterisch-flüssigkristallinen Polymeren, definiert gemäß Anspruch 1 enthalten.

9. Kosmetische und pharmazeutische Zubereitungen nach Anspruch 8, enthaltend als UV-Filter cholesterisch-flüssigkristalline Polymere in Form von Pigmenten.

10. Kosmetische und pharmazeutische Zubereitungen nach einem der Ansprüche 8 oder 9, enthaltend als UV-Filter cholesterisch-flüssigkristalline Polymere in Form von mehrschichtigen Pigmenten.

## Claims

1. The use of cholesteric liquid-crystalline, polyesters, polycarbonates or polyamides comprising
a) at least one chiral bifunctional molecular structural unit selected from the group consisting of 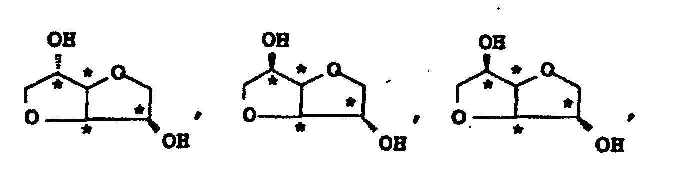 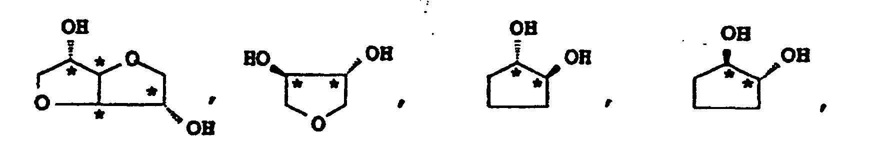 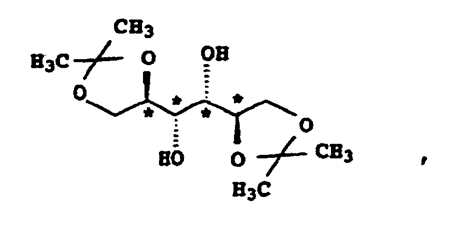 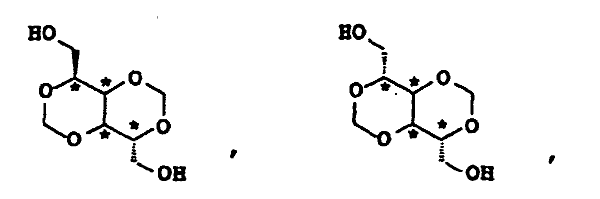 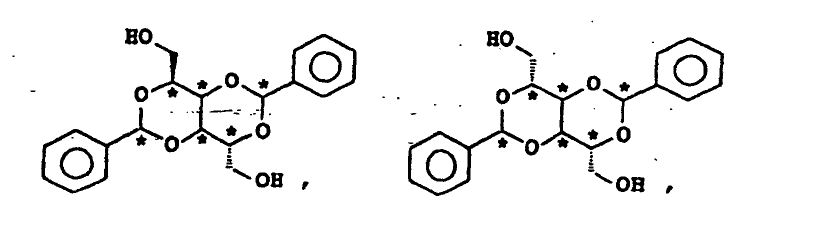 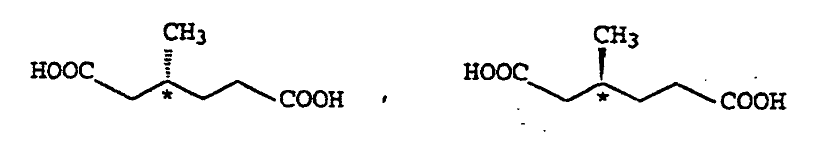 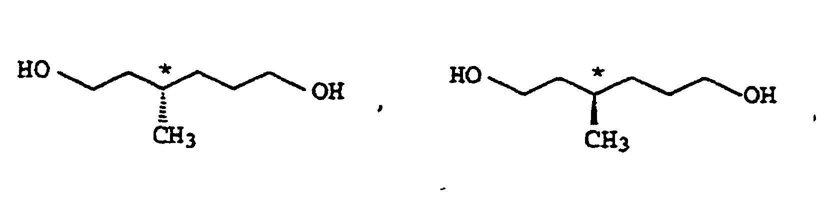 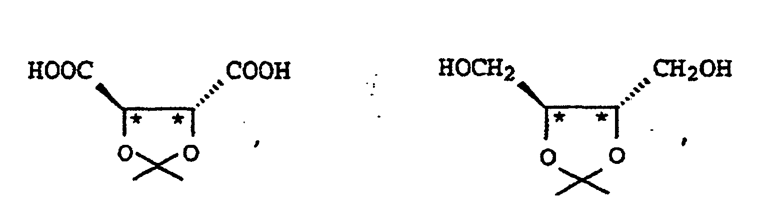 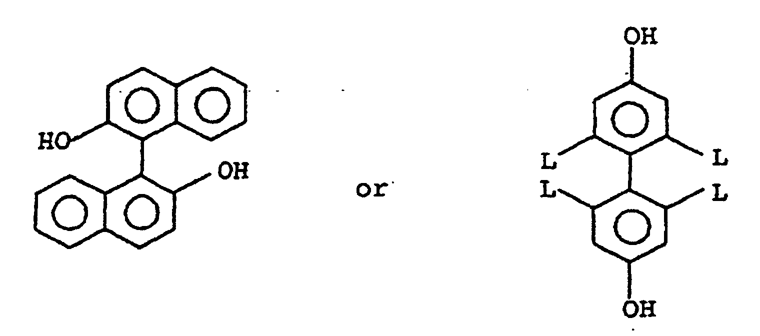 with which a cholesteric liquid-crystalline phase having a pitch of less than 450 nm can be obtained and at least one further molecular unit selected from
b) achiral compounds from the group consisting of aromatic hydroxycarboxylic acids, cycloaliphatic hydroxycarboxylic acids, aromatic aminocarboxylic acids and cycloaliphatic aminocarboxylic acids consisting of 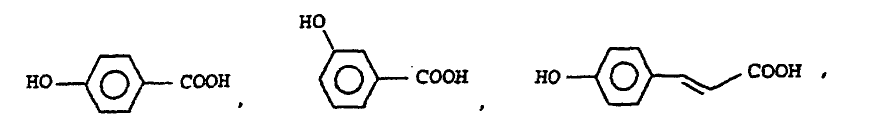 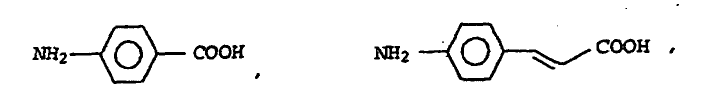 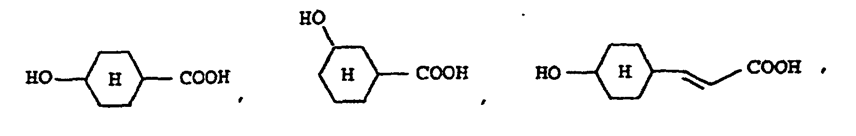 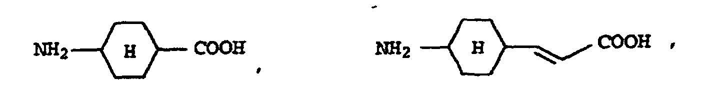 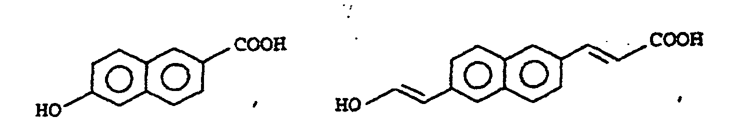 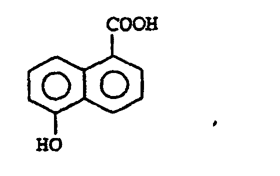 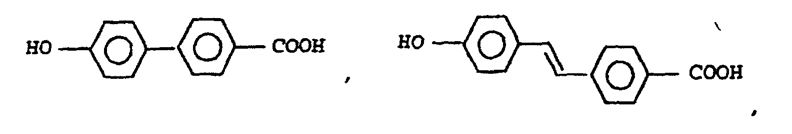 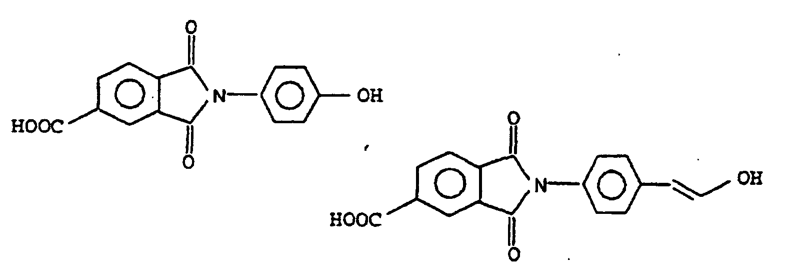
c) achiral compounds from the group consisting of aromatic dicarboxylic acids and cycloaliphatic dicarboxylic acids,
consisting of 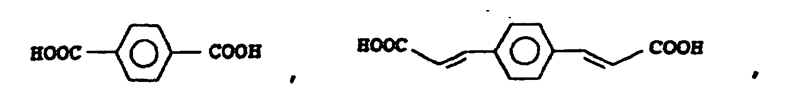 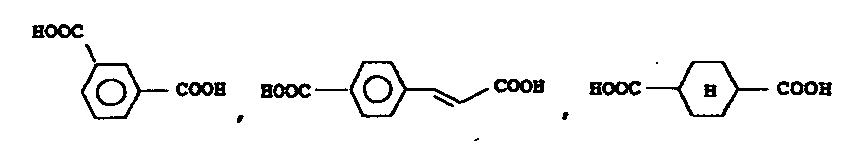 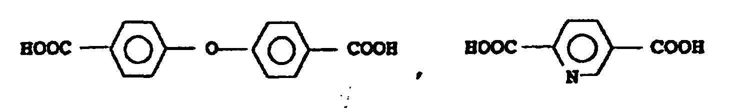 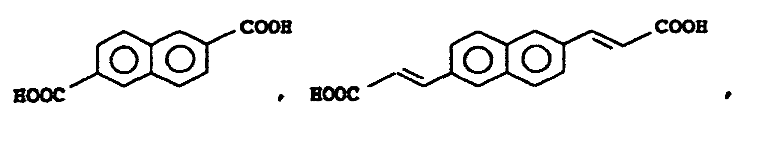 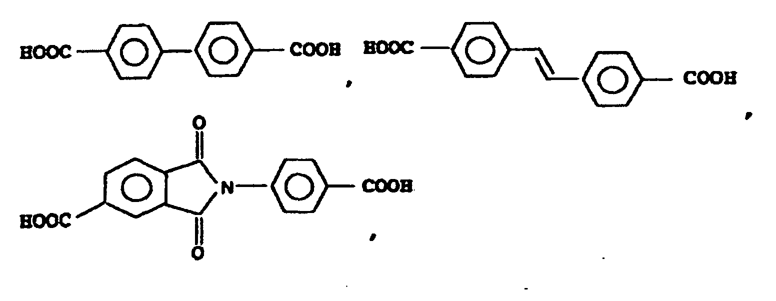 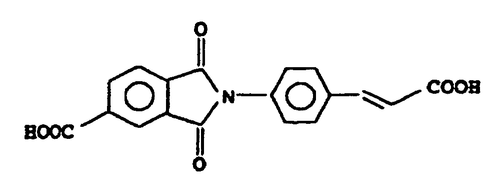 and
d) achiral compounds from the group consisting of aromatic diols, aromatic diamines and cycloaliphatic diamines consisting of 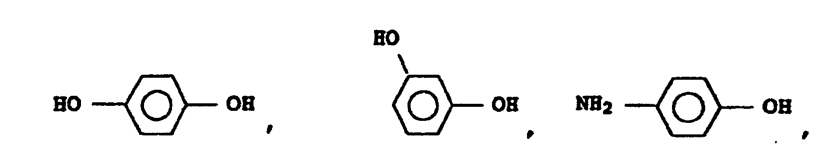 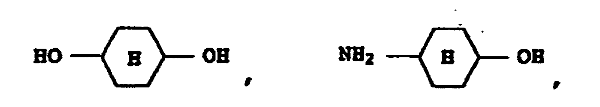 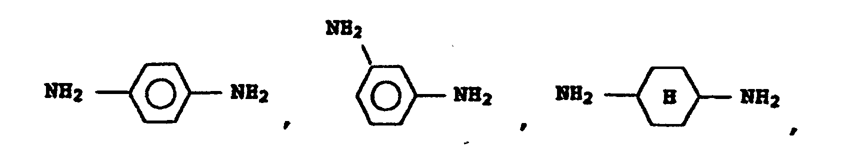 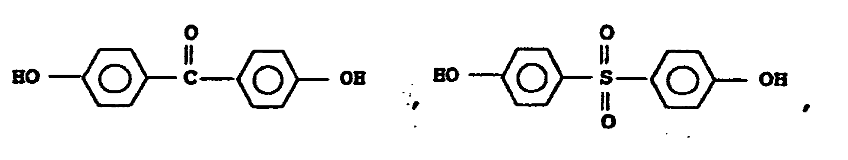 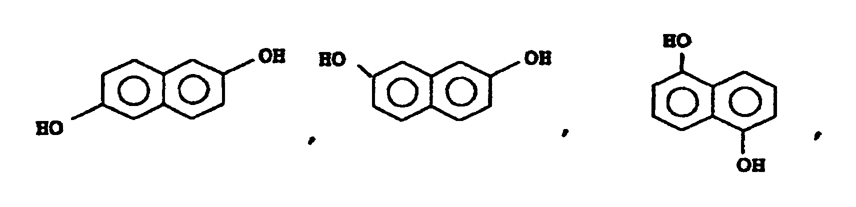 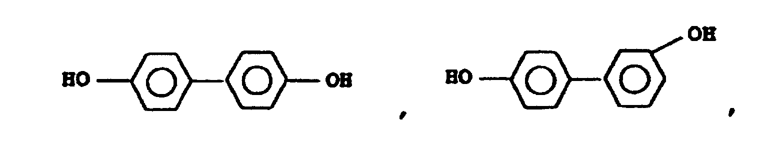 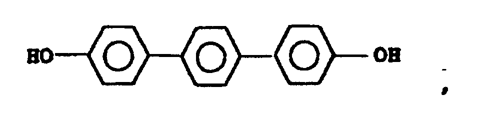 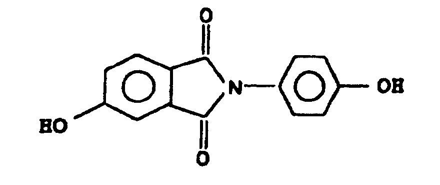 as UV screens in cosmetic and pharmaceutical preparations for protecting the human skin or hair against the sun's rays,- alone or together with UV absorbers known per se for cosmetic and pharmaceutical preparations.

2. The use of cholesteric liquid-crystalline polymers as claimed in claim 1, comprising
from 1 to 60 mol% of at least one of the chiral, bifunctional molecular units a;
from 0 to 99 mol% of at least one achiral compound b;
from 0 to 49.5 mol% of at least one achiral compound c;
from 0 to 99 mol% of at least one achiral compound d, and
from 0 to 5 mol% of dihydroxybenzoic acid or trihydroxybenzenes e,
the sum of the individual components a) to e) being 100 mol%.

3. The use of cholesteric liquid-crystalline polymers as claimed in either of claims 1 and 2 as photostable UV reflectors.

4. The use of cholesteric liquid-crystalline polymers as defined in claim 1 as UV stabilizers in cosmetic and pharmaceutical formulations.

5. The use of cholesteric liquid-crystalline polymers as claimed in any of claims 1 to 4 in the form of pigments.

6. A pigment comprising cholesteric liquid-crystalline polymers as defined in claim 1.

7. A pigment as claimed in claim 6, which is a multilayer pigment.

8. A cosmetic or pharmaceutical preparation comprising photoprotectants and intended for protecting the human epidermis or hair against UV light in the region from 280 to 400 nm, which in a cosmetically and pharmaceutically suitable carrier comprises cholesteric liquid-crystalline polymers as defined in claim 1 in amounts in which they are effective as photostable UV screens, alone or together with UV absorbers known per se for cosmetic and pharmaceutical preparations.

9. A cosmetic or pharmaceutical preparation as claimed in claim 8, comprising cholesteric liquid-crystalline polymers in the form of pigments as UV screens.

10. A cosmetic or pharmaceutical preparation as claimed in either of claims 8 and 9, comprising cholesteric liquid-crystalline polymers in the form of multilayer pigments as UV screens.

## Revendications

1. Utilisation de polyesters, polycarbonates ou polyamides à cristaux liquides cholestériques, contenant
a) au moins un constituant moléculaire bifonctionnel chiral choisi dans le groupe consistant en 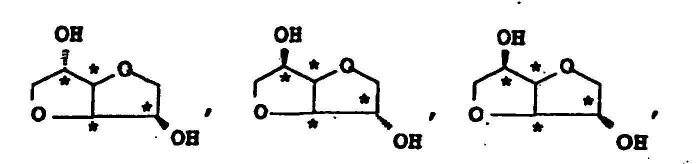 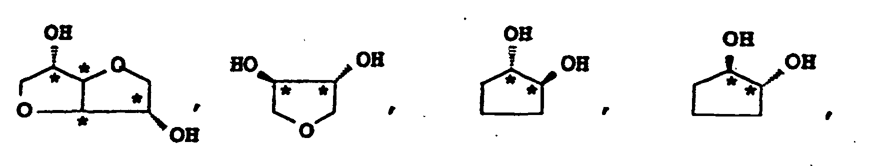 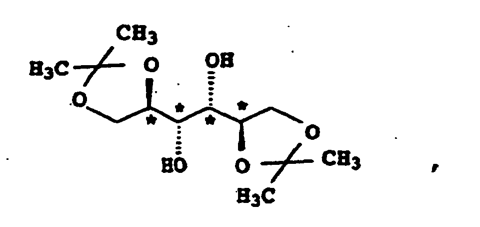 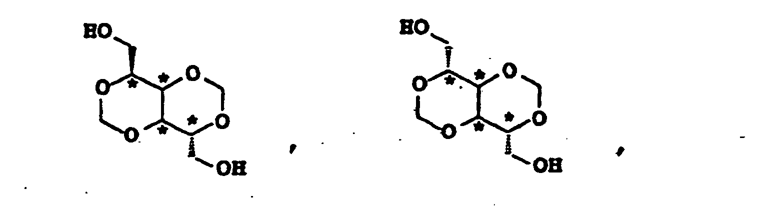 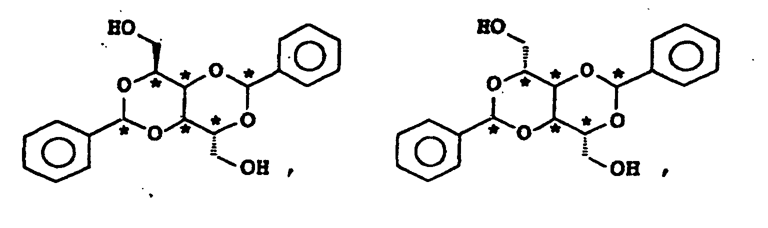 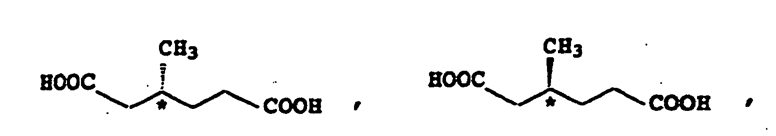 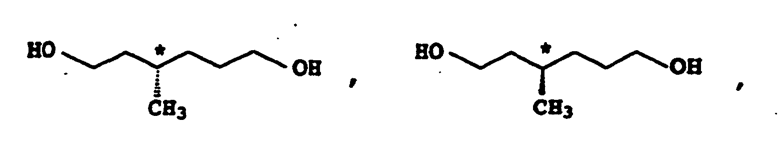 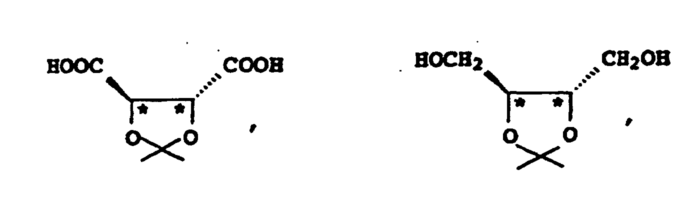 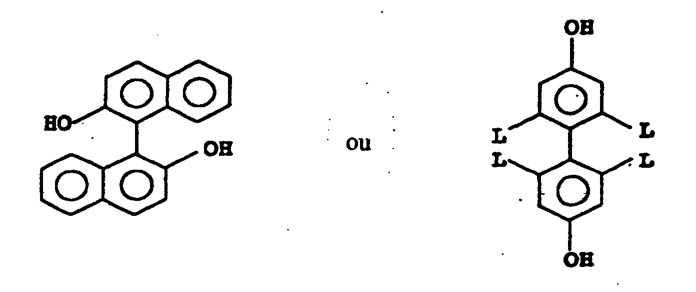 à l'aide duquel on peut parvenir à une phase à cristaux liquides cholestériques ayant un pas inférieur à 450 nm,
et au moins un autre motif moléculaire choisi parmi
b) des composés achiraux du groupe des acides hydroxycarboxyliques aromatiques, des acides hydroxycarboxyliques cycloaliphatiques, des acides aminocarboxyliques aromatiques et des acides aminocarboxyliques cycloaliphatiques, consistant en 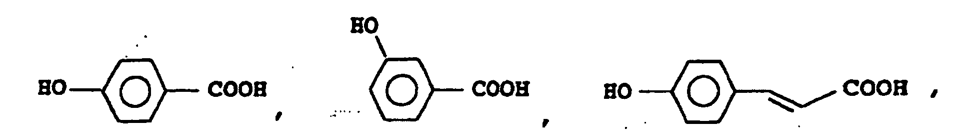 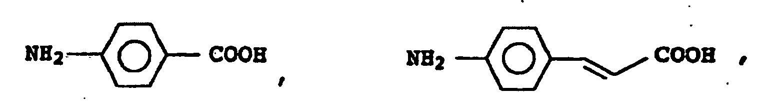 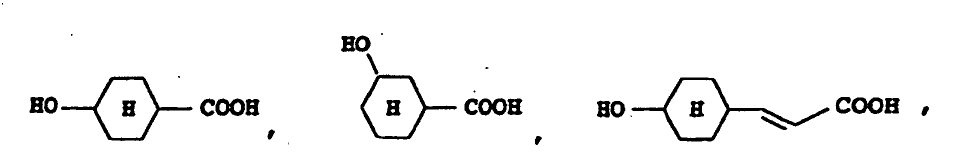 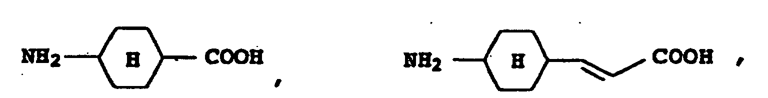 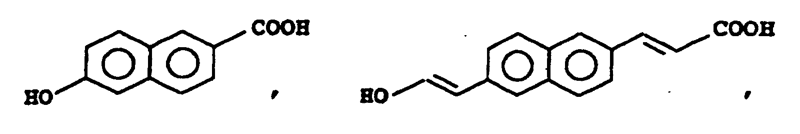 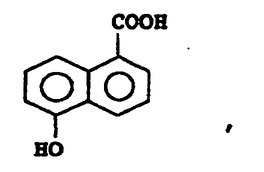 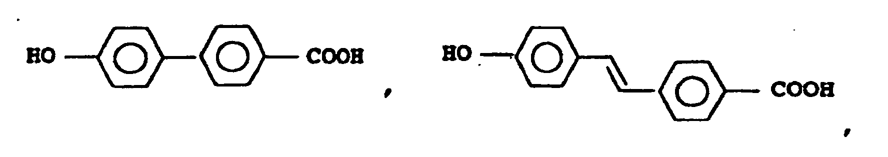 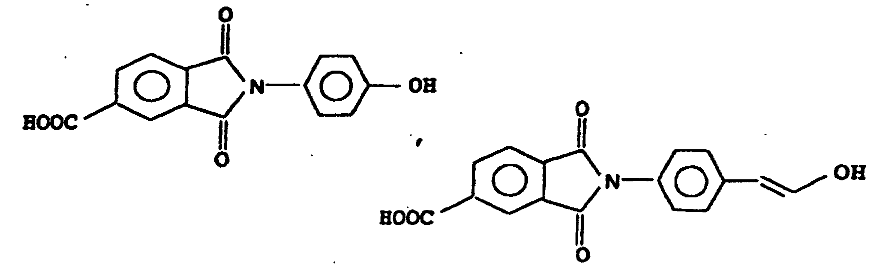
c) des composés achiraux du groupe des acides dicarboxyliques aromatiques et des acides dicarboxyliques cycloaliphatiques consistant en 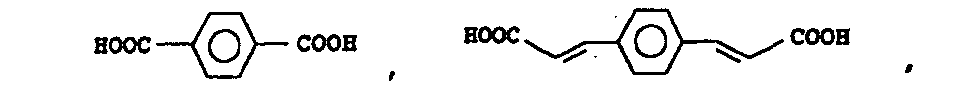 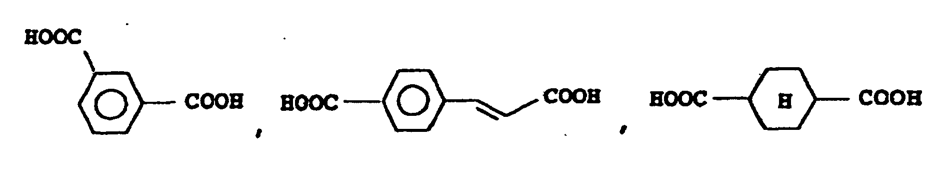 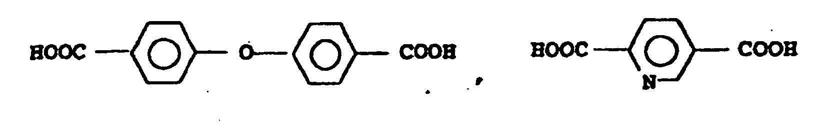 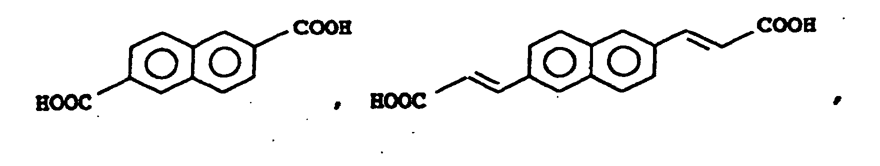 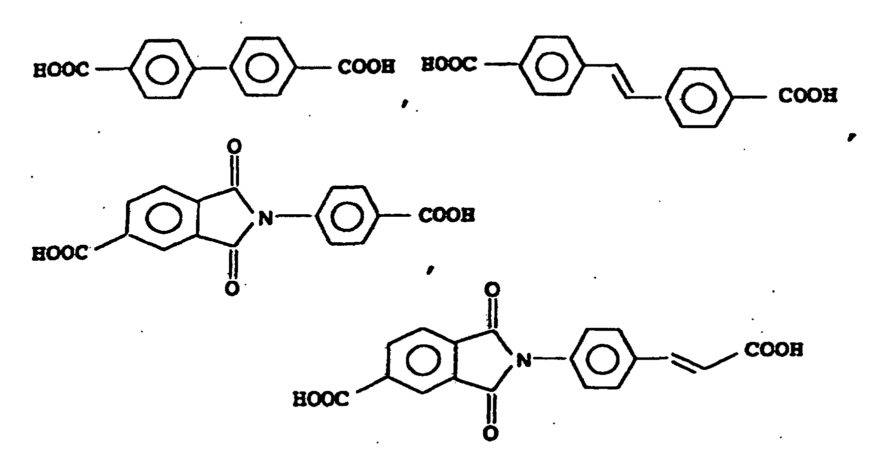 et
d) des composés achiraux du groupe des diols aromatiques, des diamines aromatiques et des diamines cycloaliphatiques consistant en 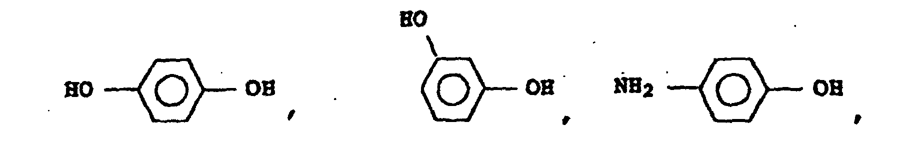 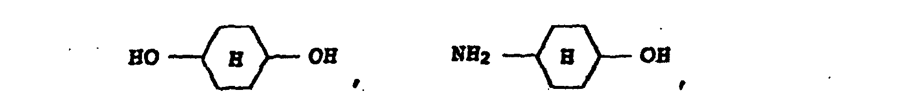 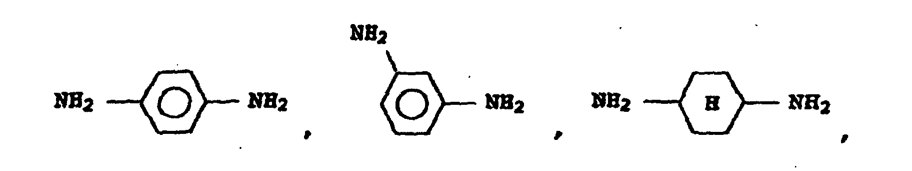 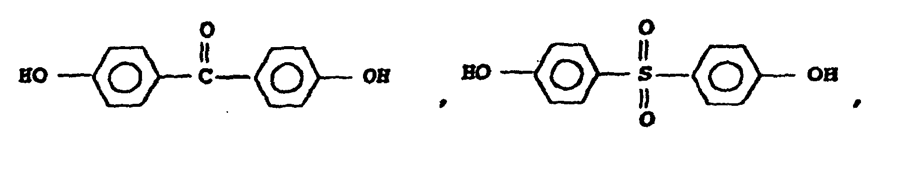 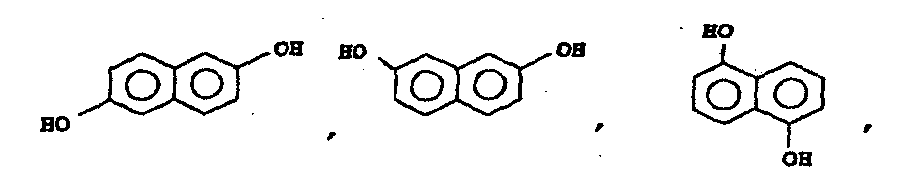 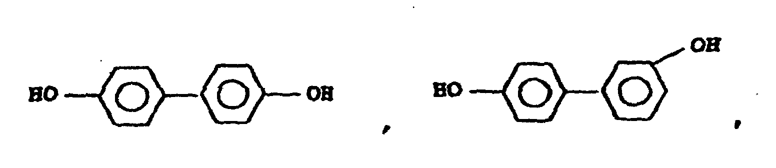 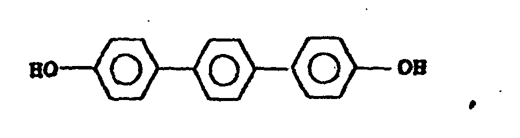 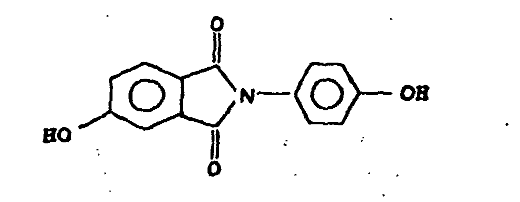 en tant que filtres UV dans des compositions cosmétiques et pharmaceutiques conçues pour la protection de la peau humaine ou de la chevelure humaine contre les rayons solaires, seuls ou avec des composés connus pour leur utilisation dans des compositions cosmétiques et pharmaceutiques et absorbant dans le domaine UV.

2. Utilisation de polymères à cristaux liquides cholestériques selon la revendication 1, contenant
1 à 60 mol % d'au moins un constituant moléculaire difonctionnel chiral a) ;
0 à 99 mol % d'au moins un composé achiral b) ;
0 à 49,5 mol % d'au moins un composé achiral c) ;
0 à 99 mol % d'au moins un composé achiral d), et
0 à 5 mol % d'acide dihydroxybenzoïque ou de trihydroxybenzènes e),
la somme des pourcentages indiqués pour les divers composants a) à e) représentant 100 mol %.

3. Utilisation de polymères à cristaux liquides cholestériques selon une des revendications 1 ou 2 en tant que réflecteurs UV photostables.

4. Utilisation de polymères à cristaux liquides cholestériques selon la revendication 1 en tant que stabilisants aux UV dans des compositions cosmétiques et pharmaceutiques.

5. Utilisation de polymères à cristaux liquides cholestériques selon une des revendications 1 à 4, sous la forme de pigments.

6. Pigments contenant des polymères à cristaux liquides cholestériques selon la revendication 1.

7. Pigments selon la revendication 6, **caractérisés en ce qu'**ils consistent en pigments lamellaires.

8. Compositions cosmétiques et pharmaceutiques contenant des agents de protection contre la lumière, conçues pour la protection de l'épiderme humain ou de la chevelure humaine contre la lumière ultra-violette de l'intervalle de 280 à 400 nm, **caractérisées en ce qu'**elles contiennent, dans un véhicule approprié à l'usage cosmétique ou pharmaceutique, seuls ou avec des composés connus pour l'utilisation dans des compositions cosmétiques et pharmaceutiques et absorbant dans le domaine UV, en tant que filtres UV photostables et en quantités efficaces, des polymères à cristaux liquides cholestériques selon la revendication 1.

9. Compositions cosmétiques et pharmaceutiques selon la revendication 8, contenant en tant que filtres UV les polymères à cristaux liquides cholestériques à l'état de pigments.

10. Compositions cosmétiques et pharmaceutiques selon une des revendications 8 ou 9, contenant en tant que filtres UV des polymères à cristaux liquides cholestériques sous forme de pigments lamellaires.
